# EUROPEAN PATENT APPLICATION

(11) **EP 1 997 834 A1**
(43) Date of publication of application: **03.12.2008**
(21) Application number: 07252186.7
(22) Date of filing: 29.05.2007
(51) Int. Cl.: C08F 4/62, C08F 10/00, C07F 7/28, C07F 7/00, C07F 5/00, C07F 11/00, C07F 19/00

(54) **Catalysts**

(71) Applicant: Borealis Technology Oy, 06101 Porvoo (FI)
(72) Inventor: Bildstein, Benno, 6020 Innsbruck (AT); Kopacka, Holger, 6200 Buch/Jenbach (AT); Enk, Barbara, 6020 Innsbruck (AT); Wurst, Klaus, 6200 Buch/Jenbach (AT); Maaranen, Janne, 04260 Kerava (FI); Vanne, Tiina, 0190 Vantaa (FI); Mansner, Erik, 00100 Helsinki (FI); Kauhanen, Jurki, 07230 Monninkyla (FI)
(74) Representative: Campbell, Neil Boyd

(57) **Abstract**

An olefin polymerisation catalyst comprising:
(I) a complex comprising a ligand of formula (I) 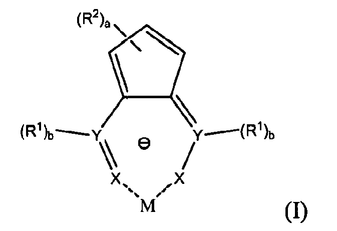 coordinated (as shown) to a metal ion M; and
(II) a cocatalyst; or the reaction product thereof;

wherein each X which may be the same or different is NR³ or O;
each Y which may be the same or different is C, S, P, S=O;
each R¹ which may be the same or different is hydrogen, an optionally substituted C₁₋₂₀ hydrocarbyl group, -CN, N(R⁴)₂, silyl, siloxy, -CF₃, -SH, -C(=NR⁴)NR⁴R⁴, -NR⁴C(=O)R⁴, -NR⁴C(=NR⁴)R⁴, -NR⁴C(=NR⁴)NR⁴R⁴, -NR⁴C(=O)OR⁴, -NR⁴C(=O)NR⁴R⁴, -NR⁴S(O)₂R⁴, -S(O)₂NR⁴R⁴, -C(=S)NR⁴R⁴, -OC(=O)R⁴, -OC(=O)NR⁴R⁴, -OR⁴, optionally substituted heterocyclyl, or optionally substituted heteroaryl;
each R² which may be the same or different is an optionally substituted C₁₋₂₀ hydrocarbyl group, halo, silyl, siloxy, nitro, -CN, N(R⁴)₂, -CF₃, -SH, C(=O)R⁴, OC(=O)R⁴, C(=O)OR⁴, -OC(=O)NR⁴R⁴, -OR⁴, optionally substituted heterocyclyl, or optionally substituted heteroaryl or two R² groups attached to adjacent ring atoms together form an optionally substituted 5- to 8-membered fused ring;
each R³ which may be the same or different is hydrogen, or an optionally substituted C₁₋₂₀ hydrocarbyl group;
each R⁴ which may be the same or different is a hydrogen, an optionally substituted C₁₋₂₀ hydrocarbyl group, or two R⁴ groups taken together form an optionally substituted 5- to 8-membered fused ring;
a is 0 to 3;
b is 1 unless the atom Y to which R¹ is attached is P wherein b is 2.

## Description

This invention relates to olefin polymerisation catalysts comprising metal complexes of multidentate ligands, as well as the use therefore in olefin polymerisation. In particular, the invention relates to olefin polymerisation catalysts which comprise gamma diketonato or gamma diketiminato ligands.

The use of metal complexes in the polymerisation of olefins is well known. Countless academic and patent publications describe the use of catalysts such as metallocenes in olefin polymerisation. Metallocenes are now used industrially and polyethylenes in particular are often produced using cyclopentadienyl based catalyst systems with all manner of different substitution patterns.

Most metallocene complexes however, are based on Π-bonding ligands such as cyclopentadienyls and indenyls in conjunction with a variety of sigma ligands, typically chlorides. The use of sigma bonding multidentate ligands, in particular bidentate ligands, in olefin polymerisation catalysts is not as common but is still described in considerable detail in the literature.

In US4,717,755, a vanadium complex of a beta-diketonato is described which can be used in the polymerisation of propylene. In US 6,034,258, a betadiketiminato complex is described in which one of the nitrogen atoms forms part of a multicyclic ring. The materials are used in ethylene polymerisation. The use therefore of multidentate ligands in the manufacture of complexes for olefin polymerisation is also known.

These betadiketonato or betaketiminate structures are attractive as on complexation with a metal ion they form a six membered ring, a highly favoured structure. Moreover, the six membered ring structure allows delocalisation to occur which makes these complexes very stable and complexes of almost all metal ions are known with beta-diketonato ligands (albeit not all for olefin polymerisation).

There remains however, a need to find new catalyst materials for olefin polymerisation as each new catalyst can impart different properties to the formed polymer and can exhibit potentially beneficial levels of activity. The present inventors have found a new class of olefin polymerisation catalysts not previously described in the art. The complexes required to form the catalysts of the invention are, *inter alia,* gamma diketonato/diketiminato type structures. Catalysts of these ligands are new and surprisingly easy to synthesis following the protocols set forth below. They form active polymerisation catalysts and can be dimerised with similar ligands to form tetradentate species.

Unlike betadiketonato or betadiketiminato type ligands, a metal ion complex of a gamma diketonato or diketiminato will form a seven membered ring. In most structures, such a seven-membered ring is not capable of supporting a delocalised electron system meaning the stability of these systems is uncertain and to date unexplored. Moreover, seven membered rings are generally not highly favoured in organic chemistry. The inventors have surprisingly found these structures can still form stable complexes with metal ions and can be used in olefin polymerisation.

No one before has made olefin polymerisation catalysts using bidentate complexes of gamma diketonato or gamma diketiminato compounds although some of the compounds necessary to form the catalysts are known in the art. The synthesis of some gamma diketofulvenes is described in JACS, 1957, vol 79, pp 4970 but no complexes thereof are suggested. Lloyd, in J Chem Soc, 1969, p 2464, reports some diacylcyclopentadienes but does not suggest that these can form complexes which can polymerise olefins.

The few disclosures of complexes of gamma diketofulvenes still do not appreciate the usefulness of these compounds in olefin polymerisation. In Calucci et al, J Organometallic Chem, 690 (2005) 4844-4855, some ruthenium and rhodium complexes of pentacarbomethoxycyclopentadiene are mentioned along side the description of various metal complexes of pentacarbomethoxycyclopentadiene, 1-benzoyl-6-hydroxy-6-phenyl fulvene and 1-benzoyl-3-nitro-6-hydroxy-6-phenyl fulvene. No mention of the use of these compounds in olefin polymerisation is made.

In Organometallics Vol 17, 1998, p3656, a diketiminato complex of the ligand 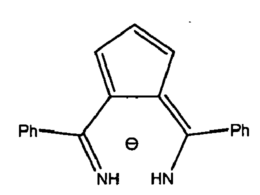 is described with magnesium or zinc. Analogous complexes in which the phenyl groups are replaced hydrogen or in which the 4-position of the cyclopentadiene ring carries trimethylsilyl are also described but are not considered as candidates for olefin polymerisation.

The present inventors have discovered that gamma diketonatos and gamma diketiminatos are useful complexes for the formation of olefin polymerisation catalysts.

Thus, viewed from one aspect the invention provides an olefin polymerisation catalyst comprising:
(I) a complex comprising a ligand of formula (I) 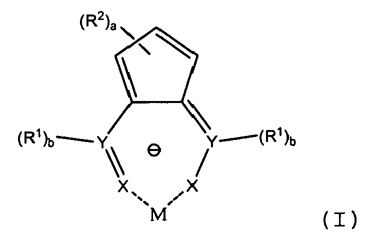 coordinated (as shown) to a metal ion M; and
(II) a cocatalyst; or the reaction product thereof;
wherein each X which may be the same or different is NR³ or O;
each Y which may be the same or different is C, S, P, S=O;
each R¹ which may be the same or different is hydrogen, an optionally substituted C₁₋₂₀ hydrocarbyl group, -CN, N(R⁴)₂, silyl, siloxy, -CF₃, -SH, -C(=NR⁴)NR⁴R⁴, -NR⁴C(=O)R⁴, -NR⁴C(=NR⁴)R⁴, -NR⁴C(=NR⁴)NR⁴R⁴, -NR⁴C(=O)OR⁴, -NR⁴C(=O)NR⁴R⁴, -NRS(O)₂R⁴, -S(O)₂NR⁴R⁴, -C(=S)NR⁴R⁴, -OC(=O)R⁴, -OC(=O)NR⁴R⁴, -OR⁴, optionally substituted heterocyclyl, or optionally substituted heteroaryl;
each R² which may be the same or different is an optionally substituted C₁₋₂₀ hydrocarbyl group, halo, silyl, siloxy, nitro, -CN, N(R⁴)₂, -CF₃, -SH, C(=O)R⁴, OC(=O)R⁴, C(=O)OR⁴, -OC(=O)NR⁴R⁴, -OR⁴, optionally substituted heterocyclyl, or optionally substituted heteroaryl or two R² groups attached to adjacent ring atoms together form an optionally substituted 5- to 8-membered fused ring;
each R³ which may be the same or different is hydrogen, or an optionally substituted C₁₋₂₀ hydrocarbyl group,
each R⁴ which may be the same or different is a hydrogen, an optionally substituted C₁₋₂₀ hydrocarbyl group, or two R⁴ groups taken together form an optionally substituted 5- to 8-membered fused ring;
a is 0 to 3;
b is 1 unless the atom Y to which R¹ is attached is P wherein b is 2.
Viewed from another aspect the invention provides an olefin polymerisation catalyst comprising:
(I) a complex comprising a metal ion coordinated by at least one gamma diketonato and/or gamma diketiminato; and
(II) a cocatalyst; or the reaction product thereof.

Viewed from another aspect the invention provides use in olefin polymerisation of a catalyst as hereinbefore defined.

Viewed from another aspect the invention provides a process for the polymerisation of at least one olefin comprising reacting said at least one olefin with a catalyst as hereinbefore described.

Various complexes of the invention are also new and form a further aspect of the invention. Viewed from another aspect therefore the invention provides a complex formed between a metal ion M and a ligand of formula (I) 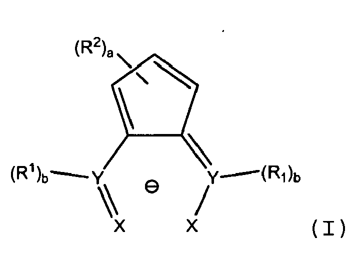 wherein R¹, R², X, Y, a and b as hereinbefore defined with the proviso that the ligand is not pentacarbomethoxycyclopentadienyl, or any of the following

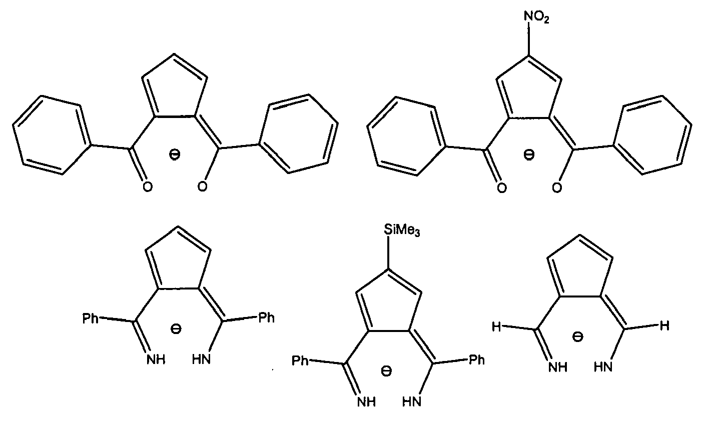

It will be appreciated that some ligands of the invention can exist in keto or enol forms. The invention covers both these forms irrespective of how a compound is actually drawn.

Throughout the description the following definitions are employed.

A gamma diketonato comprises a chelator linkage of formula -C(=O)C-C-C(=O)-. A gamma diketiminato comprises a chelator linkage of formula -C(=N)C-C-C(=N)-. It will be appreciated that only atoms necessary to form the linkage are depicted here and other atoms/bonds are required to satisfy the valency of the C and N atoms.

The term C₁₋₂₀ hydrocarbyl group, as used herein, covers any C₁₋₂₀ group comprising carbon and hydrogen only and therefore includes C₁₋₂₀ alkyl, C₂₋₂₀ alkenyl, C₂₋₂₀ alkynyl, C₃₋₂₀ cycloalkyl, C₃₋₂₀ cycloalkenyl, C₆₋₂₀ aryl groups, C₇₋₂₀ alkylaryl groups or C₇₋₂₀ arylalkyl groups. Unless otherwise stated, preferred C₁₋₂₀ hydrocarbyl groups are C₁₋₂₀ alkyl groups or C₆₋₂₀ aryl groups, especially C₁₋₈ alkyl groups or C₆₋₁₀ aryl groups. Most especially preferred groups are methyl, ethyl, propyl, isopropyl, tertbutyl, phenyl or benzyl.

The term halo includes fluoro, chloro, bromo and iodo groups, especially chloro groups.

The term silyl means a group of formula (R⁴)₃Si- where R⁴ has the meaning as hereinbefore defined. Highly preferred silyl groups are those of formula (C₁₋₆ alkyl)₃Si-, especially trimethylsilyl or tertbutyldimethylsiloxy.

The term siloxy means a group of formula (R⁴)₃SiO- where R⁴ has the meaning as hereinbefore defined. Highly preferred siloxy groups are those of formula (C₁₋₆ alkyl)₃SiO-, especially trimethylsiloxy or tertbutyldimethylsiloxy.

The term heteroaryl means a monocyclic or multicyclic aromatic ring structure comprising at least one heteroatom. Preferred heteroaryl groups have up to 20 carbon atoms, preferably up to 10 carbon atoms. Preferred heteroaryl groups have 1 to 4 heteroatoms selected from O, S and N, especially O and N. Preferred heteroaryl groups include pyridyl, pyrrolyl, furyl, indolyl, indolizinyl, benzofuranyl or benzothienyl groups.

The term heterocyclic means a monocyclic or polycyclic (non aromatic) ring structure comprising at least one heteroatom. Preferred heterocyclic groups have up to 20 carbon atoms, preferably up to 10 carbon atoms. Preferred heterocyclic groups have 1 to 3 heteroatoms selected from O, S, and N, especially O and N. Preferred heterocyclic groups include piperidinyl, furanyl, piperazinyl diazines, oxazolinyl, or thionyl.

The term optionally substituted is used herein to allow for the presence of one or more additional substituents on a group. Said optional substituents are selected from the group consisting of halo, C₁₋₂₀ hydrocarbyl, C₁₋₂₀ alkylhalide, C₁₋₂₀ alkylhydroxy, -CN, -N(R⁴)₂, silyl, oxo, siloxy, hydroxyl, -OCOC₁₋₂₀ alkyl, -NO₂, -CF₃, -SH, -C(=O)R⁴, -C(=O)OR⁴, -C(=O)NR⁴R⁴, -SR⁴, -NR⁴C(=O)R⁴, -OC(=O)R⁴, -OR⁴, heteroaryl, and heterocyclyl.

Preferred optional substituents are C₁₋₆ alkyl, NH₂, NMe₂, halo, trimethysilyl and hydroxy.

It will be appreciated that the number of optional substituents present can vary depending on the nature of the moiety carrying the optional substituents. Preferably however, 1 to 3 such optional substituents will be present, especially 1. Any optionally substituted moiety can, of course, remain unsubstituted.

The metal ion M can be any metal ion from the periodic table. Preferably the metal ion is a transition metal or lanthanide ion, especially a transition metal ion, e.g. one from groups 3 to 6 of the periodic table, Specific metal ions of interest include Sc, Y, Ti, Zr, Hf, V, Nb, Ta, Cr, Mo and W. However, the metal is preferably Sc, Y, Cr, Ti, Zr or Hf.

The oxidation state of the metal ion is governed primarily by the nature of the metal ion in question and the stability of the individual oxidation states of each metal ion. Typically, however the metal ions will be in the 3+ or 4+ oxidation state.

It will be appreciated that in the complexes of the invention, the metal ion M will also be coordinated by other ligands so as to satisfy the valency of the metal ion and to fill its available coordination sites. The nature of these further ligands can vary greatly but these will generally be η or σ-ligands.

By a σ-ligand is meant a group bonded to the metal at one or more places via a single atom, e.g. a hydrogen, halogen, silicon, carbon, oxygen, sulphur or nitrogen atom. Examples of such ligands include:
amido (e.g. NH₂)
halogenides (e.g. chloride and fluoride),
hydrogen,
triC₁₋₁₂ hydrocarbyl-silyl or -siloxy (e.g. trimethylsilyl),
triC₁₋₆hydrocarbylphosphimido (e.g. triisopropylphosphimido),
C₁₋₁₂ hydrocarbyl or hydrocarbyloxy (e.g. methyl, ethyl, phenyl, benzyl and methoxy),
diC₁₋₆ hydrocarbylamido (e.g. dimethylamido and diethylamido), and
5 to 7 ring membered heterocyclyl (e.g. pyrrolyl, furanyl and pyrrolidinyl).

Examples of particular η-ligands are well known from the technical and patent literature relating to olefin polymerization catalysts, e.g. EP-A-35242 (BASF), EP-A-129368 (Exxon), EP-A-206794 (Exxon), WO 97/28170 (Borealis), EP-A-318048, EP-A-643084, EP-A-69951, EP-A-410734, EP-A-128045, EP-B-35242 (BASF), EP-B-129368 (Exxon) and EP-B-206794 (Exxon). These include
cyclopentadienyl,
indenyl,
fluorenyl,
octahydrofluorenyl,
methylcyclopentadienyl,
1,2-dimethylcyclopentadienyl,
pentamethylcyclopentadienyl,
pentyl-cyclopentadienyl,
2-dimethyl,tertbutylsiloxy-inden-1-yl,
n-butylcyclopentadienyl,
1,3-dimethylcyclopentadienyl,
4,7-dimethylindenyl,
1-ethyl-2-methylcyclopentadienyl,
tetrahydroindenyl, and
methoxycyclopentadienyl.

Preferred ligands include an η⁵ hydrocarbyl, η¹ hydrocarbyl, halo or amido, ligands. The metal ion may also be coordinated by solvent molecules used in the manufacture of the complex e.g. THF.

In a preferred embodiment the metal ion M is also coordinated by an η⁵ hydrocarbyl ligand such as an optionally substituted cyclopentadienyl, tetrahydroindenyl or indenyl ligand. Preferred ligands of this type are cyclopentadienyl and pentamethylcyclopentadienyl. Highly preferred σ-ligands are chloro, C₁₋₆ alkyl, benzyl and amido (e.g. -NR⁴)₂.

In a still more preferred embodiment, the metal ion may be coordinated by a second and possibly third ligand of formula (I). Preferably any further ligands of formula (I) will be identical to that used to form the complex of formula (I). Highly preferred complexes of the invention are therefore of formula
(I)M(LIG)c ;
(I)₂M(LIG)c; or
(I)₃M
where (I) represents a ligand of formula (I), M represents a metal ion, both as hereinbefore defined, LIG represents a σ or η ligand (as described above) and c is 1 to 5.

It will be appreciated that the number of further ligands coordinating the metal ion M will be governed by steric issues, the number of ligands of formula (I) present and whether the complex forms in, for example, a hexagonal or trigonal bipyramidal geometry. Smaller metal ions may be more likely to adopt a trigonal bipyramidal geometry and the presence of a bulky ligand might prevent the coordination of other bulky ligands to the metal ion for steric reasons.

The skilled man can select a wide variety of ligands (LIG) which can be coordinated to the metal ion M.

In a further embodiment of the invention, a complex can comprise a ligand of formula (I) and a plurality of metal ions, typically 2 metal ions, where metal ions are bridged by sigma ligands such as halides. These complexes are new and form a still yet further aspect of the invention. Typically such complexes will comprise 2 metal ions and at least 2 ligands of formula (I).

Viewed from another aspect therefore the invention provides a complex comprising a structure of formula (VII) 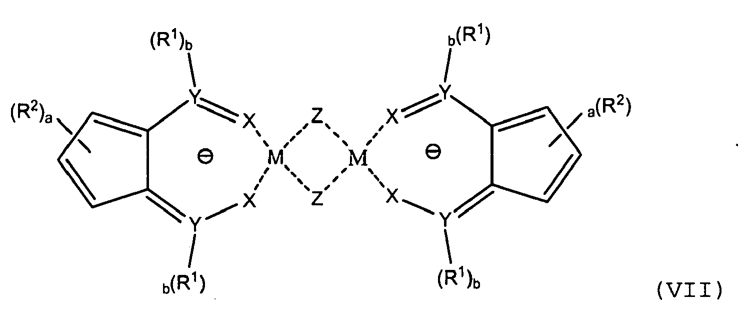 wherein R¹, R², X, Y, M, a and b as hereinbefore defined and Z represents halo. In this embodiment the metal ion M, which are preferably the same, is preferably in the 3+ oxidation state, e.g. V, Cr, Sc. It will again be appreciated that other ligands may additionally be coordinated to the metal ions, e.g. those described above.

In the ligands of the invention, the subscript "a" is preferably 0, i.e. the fulvene ring is preferably unsubstituted. If a substituent is present the subscript "a" is preferably 1 meaning 1 substituent only is present. This is preferably in the 4 position of the ring, (where positions 1 and 2 are the atoms bound to the Y atoms).

In this regard, R² is preferably a halide, especially chloride, silyl group (e.g. trimethyl silyl) or an optionally substituted C₁₋₂₀ hydrocarbyl group. More preferably R² is a C₁₋₈ alkyl group, benzyl or phenyl group. Especially preferably R² is methyl, ethyl, isopropyl, n-propyl, butyl, isobutyl or tertbutyl, most especially methyl.

In a further preferred embodiment two R² groups attached to adjacent ring atoms together form an optionally substituted 6-membered fused ring which can be saturated or more preferably unsaturated so as to form an indenyl type structure with the five membered ring.

Y is preferably a carbon atom. Preferably both Y atoms are the same. Mostly preferably they are both carbon atoms. If Y is a phosphorus atom then subscript b of the bound R¹ group is 2 to satisfy the valency of phosphorus (V). For all other Y atoms, b is 1.

X is O or NR³. Preferably both X atoms are the same.

R³ is preferably a C₁₋₂₀ hydrocarbyl group, more preferably a C₆₋₂₀ aryl group or C₇₋₂₀ arylalkyl group. Especially preferably R³ is phenyl or diC₁₋₆alkylphenyl.

Each R¹ may be the same or different but preferably the R¹ groups are the same. R¹ is preferably hydrogen, an optionally substituted C₁₋₂₀ hydrocarbyl group or N(R⁴)₂ group. More preferably R¹ is an optionally substituted C₁₋₂₀ alkyl, optionally substituted C₃₋₂₀ cycloalkyl or optionally substituted C₆₋₂₀ aryl group, most especially an optionally substituted aryl group, e.g. phenyl.

R⁴ is preferably hydrogen or a C₁₋₂₀ hydrocarbyl group, more preferably a C₁₋₆ alkyl group. Especially preferably R⁴ is methyl.

Preferred complexes of the invention include those of a metal ion with a ligand of formula (II) 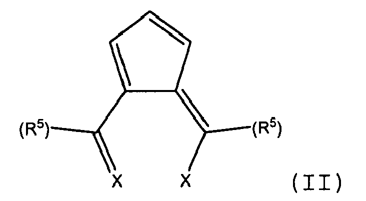 where R⁵ is an optionally substituted phenyl group and X is as hereinbefore defined.

Other preferred complexes of the invention, which are novel in their own right, include those of a metal ion with a ligand of formula (III) 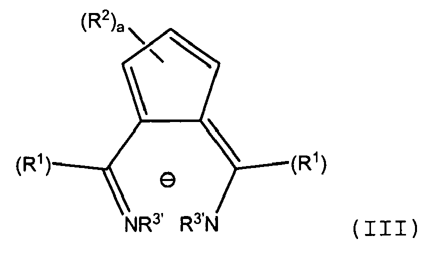 where R¹, R², and a are as hereinbefore described and R³' is as hereinbefore described for R³ but does not represent hydrogen.

Still more preferred complexes are those of a metal ion with a ligand of formula (III') 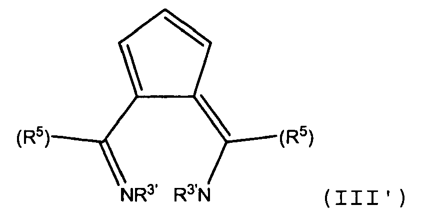 wherein R^{3'} and R⁵ are as hereinbefore described.

In a further preferred embodiment of the invention, ligands of the invention can be dimerised to make tetradentate ligands which are also capable of coordinating metal ions to form complexes which can be used in olefin polymerisation reactions upon appropriate reaction with a cocatalyst.

Viewed from a still further aspect therefore the invention provides a complex comprising a metal ion M coordinated to a ligand of formula (IV). 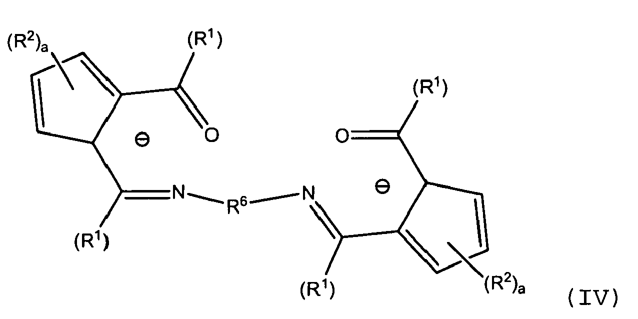 where R¹, R², and a are as hereinbefore defined and R⁶ is a linking group, e.g. an alkylene chain.

Preferably R⁶ is an ethylene linker.

The invention also encompasses catalysts made by the reaction of such a complex with a cocatalyst and the use of such a catalyst in an olefin polymerisation process.

Throughout the disclosure above, where a narrower definition of a substituent is presented, that narrower definition is deemed disclosed in conjunction with all broader and narrower definitions of other substituents in the application as well.

### Synthesis

The complexes of the invention can be synthesised by any process and the skilled organic chemist would be able to devise various synthetic protocols for the manufacture of the necessary ligand materials. The chemistry involved can however, be difficult in view of the presence of the heteroatoms and in view of the complexity of the molecules themselves. Ideally however, the process should be simple and quick. The inventors have found an ideal process for the manufacture of gamma diketonato ligands based on JACS 1957, 79, 4970.

The specific nature of the process obviously depends on the nature of the target ligand but the process typically requires cyclopentadiene or an analogue thereof as a starting material. This can be deprotonated using any convenient base such as butyl lithium or sodium hydroxide to form cyclopentadienide. It will be appreciated that should it be desired that the cyclopentadiene structure comprise substituents (R²) then these are most conveniently introduced before deprotonation using known chemistry. The nature of the base used chosen for deprotonation is one which would not affect any substituents present. An unsubstituted cyclopentadienide is the preferred choice however.

The cyclopentadienide may then be reacted with an activated carbonyl compound such as benzoyl chloride. By activated is meant that the carbonyl is attached to a leaving group. Activated carbonyls therefore include acid chlorides, anhydrides, esters and the like.

The nature of the rest of the activated carbonyl compound will of course depend on the nature of the desired end ligand, i.e. the nature of the R¹ substituent. Typically however the molecule will comprise an optionally substituted aromatic group, e.g. phenyl. These reactions are summarised in scheme 1 based on the reaction of cyclopentadienide with benzoyl chloride:
**Scheme 1**

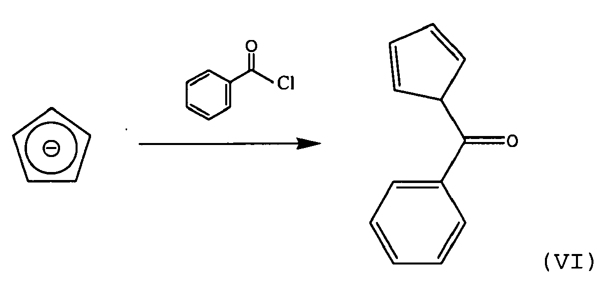

The formed compound (VI) has only one heteroatom and it is therefore necessary for the cyclopentadiene structure to react again with further activated carbonyl compound to introduce a second heteroatom into the molecule. This could obviously be achieved by contacting the intermediate product (VI) with more base and then more activated carbonyl compound (or even a different activated carbonyl compound) but such a process is laborious and time consuming.

In fact, the entire reaction can be effected in a single reaction step as the intermediate compound (VI) is more acidic than cyclopentadienide. This means that excess cyclopentadienide will deprotonate the intermediate compound (VI) forming cyclopentadiene as a byproduct but also forming an anion which can react with more activated carbonyl compound. The formation of a gamma diketonato ligands of the invention can therefore be effected in one step and thus in one reaction vessel if the amounts of cyclopentadienide and activated compound are in fact around equimolar. In effect, there will be 2 equivalents of activated compound present, 1 equivalent of cyclopentadienide for reaction and 1 equivalent acting as a base. These reactions are summarised in scheme 2 based on the reaction product of scheme 1 and benzoyl chloride.
**Scheme 2**

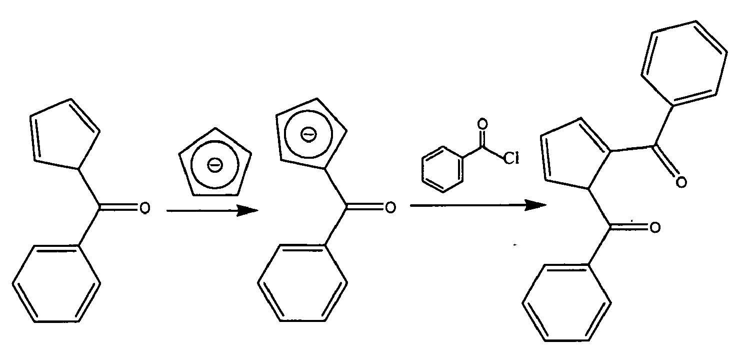

It will be appreciated that a diketonato of this structure is in equilibrium with its enol form and may well exist predominatly in that enol form. Where a compound is depicted as a diketonato that structure is deemed also to cover the enol form of that material.

The activated compound with which the cyclopentadienide material reacts can vary considerably. As well as compounds like benzoyl chloride, i.e. those of general formula RCOLG (where LG is a leaving group), the activated material may be based on a carbamoyl structure (R₂NHCOLG) or potentially based on a RSO-LG, RSO₂-LG or R₂PO-LG type structure.

The formed ligand can then be complexed as described more fully below but the ligand could be reacted further to introduce more substituents of interest. Also, the ligands could be dimerised to form larger higher dentate species. Such ligands are called salen type ligands. In one embodiment therefore, a gamma diketonato ligand can be reacted with an bisimide forming agent such as ethylene diamine to dimerise two ligands. The amine groups of the agent will react with carbonyl groups of the gamma diketonato ligands forming a dimerised species. The result is a tetradentate ligand of great versitility and this reaction is summarised in scheme 3 below.
**Scheme 3**

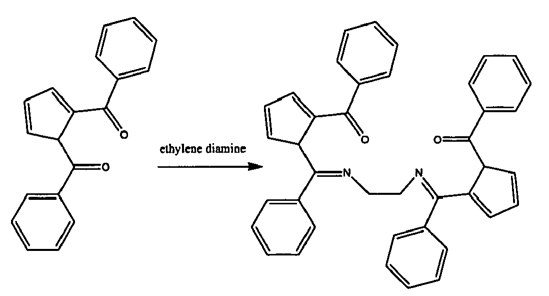

This reaction forms a further aspect of the invention which therefore provides a process for the preparation of a tetradentate ligand comprising reacting a compound of formula (VII) 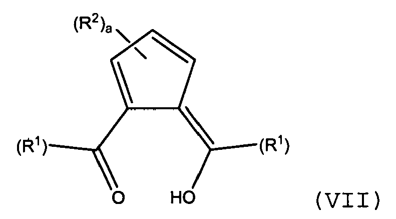 wherein R¹, R² and a are as hereinbefore defined;
with a compound of formula NH₂R⁶NH₂ wherein R⁶ is a linking group.

The invention also covers ligands comprising aminoiminofulvene structures. Aminoiminofulvenes are known in the art but their synthesis is laborious and slow. In JACS, 1970, 92, 4849, Muller-Westerhof reports the synthesis of 6-phenylamino-2-phenyliminofulvene by reaction of 6-dimethylaminofulvene with oxalyl chloride and subsequent transamination (twice) with aniline (see scheme 4)
**Scheme 4**

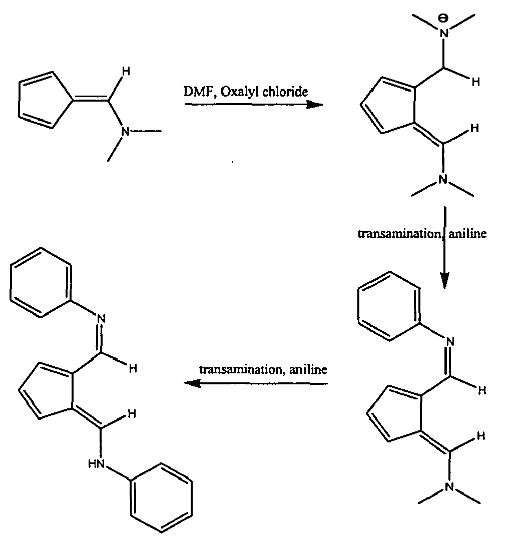

This multistage process is unsatisfactory for many reasons so the inventors sought an alternative process for the manufacture of ligands of the invention. The inventors have realised that a process similar to that used to manufacture gamma diketonatos can also be used to manufacture gamma diketiminatos (i.e. aminoiminofulvenes). Thus, cyclopentadienide can react with an imide of formula RC=N(R)LG following a similar mechanism to that described above for gamma diketonatos. The leaving group LG can be a chloride. This reaction is new and forms a still further aspect of the invention.

Thus, viewed from another aspect the invention provides a process in which a compound of formula 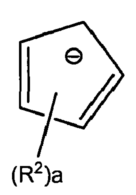 is combined with a compound 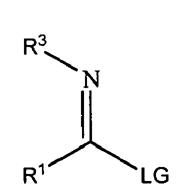 where LG is a leaving group, e.g. chloro, so as to form a compound

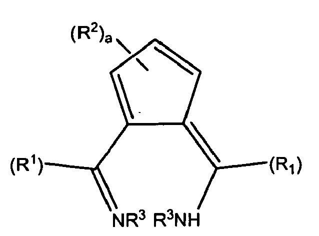

As above, it is preferred if the amount of each reactant is approximately equimolar, e.g. within 10 mol% of equimolar.

The starting imide material can vary a great deal allowing the skilled man great flexibility in the type of imide compound he uses and hence the type of functional groups which are eventually incorporated into the ligand. Imido synthons can be prepared using literature techniques described for example in Boere, J Chem Soc Dalton Trans 1998, 4147. Here an optionally substituted aniline is reacted with a benzoyl chloride type compound in the presence of sodium carbonate to form an N-arylbenzamide intermediate which reacts with SOCl₂ to give an N-arylbenzimidoyl chloride. This chemistry is summarised below in scheme 5 and the reaction allows the formation of many different starting imides for use in the formation of the ligands of the invention.
**Scheme 5**

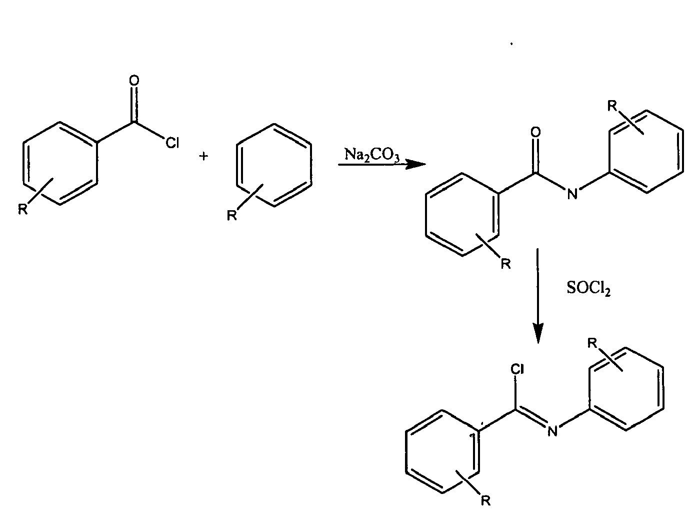

Formation of the desired complex is effected by reacting the desired ligand with an appropriate quantity of base, e.g. an organolithium compound, such as methyllithium or butyllithium.

The ligand can then be metallated conventionally, e.g. by reaction with a halide of the metal, preferably in an organic solvent, e.g. a hydrocarbon or a hydrocarbon/ ether mixture or ether (THF). The metal halide can, of course, contain substituents other than halides such as cyclopentadienyl ligands. The ligand could be reacted therefore with a compound MCl₃, MCl₄ or CpMCl₃.

An alternative approach to the complexes is also envisaged where the deprotonated fulvene is reacted with M(NMe₂)₄ or M(CH₂Ph)₄. The resulting complexes may therefore contain amino or benzyl sigma ligands rather than halide ligands. In essence, as long as their are groups to displace, any metal compound can be used to form the complexes of the invention depending on the nature of any other ligands which the skilled man wants to be present.

σ-ligands other than chlorine may, however, also be introduced by displacement of chlorine from a complex metal chloride by reaction with appropriate nucleophilic reagent (e.g. methyl lithium or methylmagnesium chloride) or using, instead of a metal halide, a reagent such as tetrakisdimethylamidotitanium or metal compounds with mixed chloro and dimethylamido ligands.

### Catalysts

To form an active catalytic species it is necessary to employ a cocatalyst as is well known in the art. Cocatalysts used to activate metallocene catalysts are suitable for use in this invention. Complex and cocatalyst may be introduced into the polymerization reactor separately or together or, more preferably they are pre-reacted and their reaction product is introduced into the polymerization reactor.

As mentioned above, the olefin polymerisation catalyst system of the invention comprises (i) a complex in which the metal ion is coordinated by a ligand of the invention; and normally (ii) an aluminium alkyl compound (or other appropriate cocatalyst), or the reaction product thereof.

While the aluminium alkyl compound may be an aluminium trialkyl (e.g. triethylaluminium (TEA)) or an aluminium dialkyl halide (e.g. diethyl aluminium chloride (DEAC)), it is preferably an alumoxane, either MAO or an alumoxane other than MAO, such as an isobutylalumoxane, e.g. TIBAO (tetraisobutylalumoxane) or HIBAO (hexaisobutylalumoxane). Alternatively, however, the alkylated (e.g. methylated) catalysts of the invention may be used with other cocatalysts, e.g. boron compounds such as B(C₆F₅)₃, C₆H₅N(CH₃)₂H:B(C₆F₅)₄, (C₆H₅)₃C:B(C₆F₅)₄ or Ni(CN)₄[B(C₆F₅)₃]₄²⁻.

However, when the metal in the catalyst is a group 3 transition metal, i.e. Sc, Y, La or Ac, no co-activator is not necessarily required since such catalyst species are already in an active form.

If desired the complex, complex/cocatalyst mixture or a complex/cocatalyst reaction product may be used in unsupported form, i.e. complex and MAO can be precipitated without an actual carrier material and used as such. However the complex or its reaction product with the cocatalyst is preferably introduced into the polymerization reactor in supported form, e.g. impregnated into a porous particulate support.

The particulate support material used is preferably an organic or inorganic material, e.g. a polymer (such as for example polyethylene, polypropylene, an ethylene-propylene copolymer, another polyolefin or polystyrene or a combination thereof). Such polymeric supports may be formed by precipitating a polymer or by a prepolymerization, e.g. of monomers used in the polymerization for which the catalyst is intended. However, the support is especially preferably a metal or metalloid oxide such as silica, alumina or zirconia or a mixed oxide such as silica-alumina, in particular silica, alumina or silica-alumina.

Particularly preferably, the support material is acidic, e.g. having an acidity greater than or equal to silica, more preferably greater than or equal to silica-alumina and even more preferably greater than or equal to alumina. The acidity of the support material can be studied and compared using the TPD (temperature programmed desorption of gas) method. Generally the gas used will be ammonia. The more acidic the support, the higher will be its capacity to adsorb ammonia gas. After being saturated with ammonia, the sample of support material is heated in a controlled fashion and the quantity of ammonia desorbed is measured as a function of temperature.

Especially preferably the support is a porous material so that the complex may be loaded into the pores of the support, e.g. using a process analogous to those described in WO94/14856 (Mobil), WO95/12622 (Borealis) and WO96/00243 (Exxon). The particle size is not critical but is preferably in the range 5 to 200 µm, more preferably 20 to 80 µm.

Before loading, the particulate support material is preferably calcined, i.e. heat treated, preferably under a non-reactive gas such as nitrogen. This treatment is preferably at a temperature in excess of 100°C, more preferably 200°C or higher, e.g. 200-800°C, particularly about 300°C. The calcination treatment is preferably effected for several hours, e.g. 2 to 30 hours, more preferably about 10 hours.

The support may be treated with an alkylating agent before being loaded with the catalyst. Treatment with the alkylating agent may be effected using an alkylating agent in a gas or liquid phase, e.g. in an organic solvent for the alkylating agent. The alkylating agent may be any agent capable of introducing alkyl groups, preferably C₁₋₆ alkyl groups and most especially preferably methyl groups. Such agents are well known in the field of synthetic organic chemistry. Preferably the alkylating agent is an organometallic compound, especially an organoaluminium compound (such as trimethylaluminium (TMA), dimethyl aluminium chloride, triethylaluminium) or a compound such as methyl lithium, dimethyl magnesium, triethylboron, etc.

The quantity of alkylating agent used will depend upon the number of active sites on the surface of the carrier. Thus for example, for a silica support, surface hydroxyls are capable of reacting with the alkylating agent. In general, an excess of alkylating agent is preferably used with any unreacted alkylating agent subsequently being washed away.

Following treatment of the support material with the alkylating agent, the support is preferably removed from the treatment fluid and any excess treatment fluid is allowed to drain off.

The optionally alkylated support material is loaded with the catalyst. This loading may be effected by using a solution of the catalyst in an organic solvent therefor, e.g. as described in the patent publications referred to above. Preferably, the volume of catalyst solution used is from 50 to 500% of the pore volume of the carrier, more especially preferably 80 to 120%. The concentration of catalyst compound in the solution used can vary from dilute to saturated depending on the amount of metallocene active sites that it is desired be loaded into the carrier pores.

The active metal (i.e. the metal of the catalyst) is preferably loaded onto the support material at from 0.1 to 4%, preferably 0.5 to 3.0%, especially 1.0 to 2.0%, by weight metal relative to the dry weight of the support material.

After loading of the catalyst onto the support material, the loaded support may be recovered for use in olefin polymerization, e.g. by separation of any excess catalyst solution and if desired drying of the loaded support, optionally at elevated temperatures, e.g. 25 to 80°C.

Alternatively, a cocatalyst, e.g. an alumoxane or an ionic catalyst activator (such as a boron or aluminium compound, especially a fluoroborate) may also be mixed with or loaded onto the catalyst support material. This may be done subsequently or more preferably simultaneously to loading of the complex, for example by including the cocatalyst in the solution of the catalyst, by contacting the catalyst loaded support material with a solution of the cocatalyst or catalyst activator, e.g. a solution in an organic solvent, or by first impregnating the cocatalyst with a support and then contacting the cocatalyst impregnated support with a solution of the catalyst or neat catalyst (e.g. as described in W096/32423). Alternatively however any such further material may be added to the catalyst-loaded support material in the polymerization reactor or shortly before dosing of the catalyst material into the reactor.

In this regard, as an alternative to an alumoxane it may be preferred to use a fluoroborate catalyst activator for the alkylated catalysts, especially a B(C₆F₅)₃ or more especially a-B(C₆F₅)₄ compound, such as C₆H₅N(CH₃)₂H:B(C₆F₅)₄ or (C₆H₅)₃C:B(C₆F₅)₄. Other borates of general formula (cation)ₐ (borate)_{b} where a and b are positive numbers, may also be used.

As an alternative to the loading of the optionally alkylated support material with a solution of the procatalyst in an organic solvent, loading of the catalyst may be effected by mixing it with the optionally alkylated support material in the absence of solvents with said carrier at a temperature of at least 50°C but less than the vaporisation temperature of the metallocene compound. The particular features of this so-called dry mixing method are disclosed in WO 96/32423 (Borealis). If use of a cocatalyst/catalyst activator in such process is desired, this may be impregnated into the optionally alkylated support material prior to loading of the catalyst.

Where such a cocatalyst or catalyst activator is used, it is preferably used in a mole ratio to the metallocene of from 0.1:1 to 10000:1, especially 1:1 to 50:1, particularly 1:2 to 30:1. More particularly, where an alumoxane cocatalyst is used, then for an unsupported catalyst the aluminium:metallocene metal (M) molar ratio is conveniently 2:1 to 10000:1, preferably 50:1 to 1000:1. Where the catalyst is supported the A1:M molar ratio is conveniently 2:1 to 10000:1 preferably 50:1 to 400:1. Where a borane cocatalyst (catalyst activator) is used, the B:M molar ratio is conveniently 2:1 to 1:2, preferably 9:10 to 10:9, especially 1:1. When a neutral triarylboron type cocatalyst is used the B:M molar ratio is typically 1:2 to 500:1, however some aluminium alkyl would normally also be used. When using ionic tetraaryl borate compounds, it is preferred to use carbonium rather than ammonium counterions or to use B:M molar ratio 1:1.

Where the further material is loaded onto the catalyst loaded support material, the support may be recovered and if desired dried before use in olefin polymerization.

The olefin polymerized in the process of the invention is preferably ethylene or an alpha-olefin or a mixture of ethylene and an α-olefin or a mixture of alpha olefins, for example C₂₋₂₀ olefins, e.g. ethylene, propene, 1-butene, 1-hexene, 4-methyl-1-pentene, 1-octene etc. The olefins polymerized in the method of the invention may include any compound which includes unsaturated polymerizable groups. Thus for example unsaturated compounds, such as C₆₋₂₀ olefins (including cyclic and polycyclic olefins (e.g. norbornene)), and polyenes, especially C₄₋₂₀ dienes, may be included in a comonomer mixture with lower olefins, e.g. C₂₋₅ α-olefins. Diolefins (i.e. dienes) are suitably used for introducing long chain branching into the resultant polymer. Examples of such dienes include α,ω linear dienes such as 1,5-hexadiene, 1,6-heptadiene, 1,8-nonadiene, 1,9-decadiene, etc.

In general, where the polymer being produced is a homopolymer it will preferably be polyethylene or polypropylene. Where the polymer being produced is a copolymer it will likewise preferably be an ethylene or propylene copolymer with ethylene or propylene making up the major proportion (by number and more preferably by weight) of the monomer residues. Comonomers, such as C₄₋₆ alkenes, will generally be incorporated to contribute to the mechanical strength of the polymer product.

Polymerization in the method of the invention may be effected in one or more, e.g. 1, 2 or 3, polymerization reactors, using conventional polymerization techniques, e.g. gas phase, solution phase, slurry or bulk polymerization.

In general, a combination of slurry (or bulk) and at least one gas phase reactor is often preferred, particularly with the reactor order being slurry (or bulk) then one or more gas phase.

For slurry reactors, the reaction temperature will generally be in the range 60 to 110°C (e.g. 85-110°C), the reactor pressure will generally be in the range 5 to 80 bar (e.g. 50-65 bar), and the residence time will generally be in the range 0.3 to 5 hours (e.g. 0.5 to 2 hours). The diluent used will generally be an aliphatic hydrocarbon having a boiling point in the range -70 to +100°C. In such reactors, polymerization may if desired be effected under supercritical conditions.

For gas phase reactors, the reaction temperature used will generally be in the range 60 to 115°C (e.g. 70 to 110°C), the reactor pressure will generally be in the range 10 to 25 bar, and the residence time will generally be 1 to 8 hours. The gas used will commonly be a non-reactive gas such as nitrogen together with monomer (e.g. ethylene).

For solution phase reactors, the reaction temperature used will generally be in the range 130 to 270°C, the reactor pressure will generally be in the range 20 to 400 bar and the residence time will generally be in the range 0.005 to 1 hour. The solvent used will commonly be a hydrocarbon with a boiling point in the range 80-200°C.

Generally the quantity of catalyst used will depend upon the nature of the catalyst, the reactor types and conditions and the properties desired for the polymer product. Conventional catalyst quantities, such as described in the publications referred to herein, may be used.

The invention will now be illustrated by reference to the following nonlimiting Examples:

### Experimental

For the synthetic part, the following instrumentation was used:
NMR spectroscopy: Bruker DPX 300 NMR spectrometer
IR spectroscopy: Nicolet 5700 FT-IR spectrometer equipped with a Smart Orbit diamond ATR unit
Single crystal structure analysis: Nonius Kappa-CCD equipped with graphite-monochromated Mo-K(alpha) radiation

### Ligand and complex synthesis

### Example 1

### Ligand 1: 2-Benzoyl-6-hydroxy-6-phenylfulvene

Modified and improved preparation according to: (a) W.J. Linn, W.H. Sharkey, J. Am. Chem. Soc. 79 (1957) 4970; (b) D. Lloyd, N.W. Preston, J. Chem. Soc. (C) (1969) 2464; (c) W.F. Little, R. Koestler, J. Org. Chem. 26 (1961) 3246. 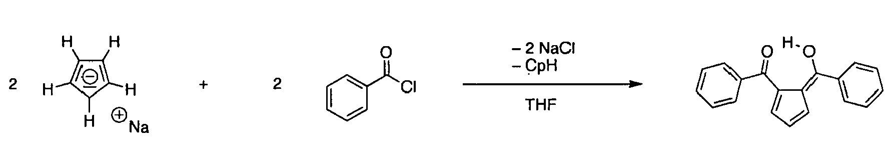 A Schlenk vessel was charged with 10 ml tetrahydrofuran (THF) and 8.0 ml (16 mmol) of a 2.0 molar solution of sodium cyclopentadienide in THF. After the mixture was cooled to -30 °C, a solution of 1.9 ml (16 mmol) benzoyl chloride in 20 ml THF was added dropwise to the stirred mixture during a period of 30 minutes. The cooling bath was removed and the mixture was allowed to warm to room temperature under further stirring. After 2 hours, the yellow solution was hydrolyzed by addition of ice.

Workup: THF was removed on a rotary evaporator, the residue was extracted with three portions of diethyl ether to remove organic byproducts. The combined yellow aqueous layers were neutralized by addition of appropriate portions of diluted hydrochloric acid, resulting in precipitation of the crude product. The product was extracted into diethyl ether, the combined organic layers were dried with anhydrous sodium sulfate, and the solution was concentrated on a rotary evaporator to a volume of approximately 5 ml. After layering this solution with n-hexane, the pure product crystallized in the form of large crystals in 55% yield (1.20 g).
m.p.: 102 °C. 1H-NMR (CD₂Cl₂): 6.53 (t, 1H, J = 4 Hz), 7.30 (d, 2H, J = 4 Hz), 7.51-7.65 (m, 6H), 7.81-7.84 (m, 4H), enol-H not observed, ppm. 13C-NMR (CD₂Cl₂): 123.4, 124.6, 128.5, 130.0, 131.7, 137.9, 142.0, 185.6 ppm. MS (FAB pos): 275.05 (M+H)+ m/z. IR (ATR): 574.2 s, 699.4 s, 738.3 m, 760.5 s, 785.2 s, 871.8 w, 1024.0 w, 1075.8 m, 1086.9 m, 1135.0 m, 1280.2 w, 1362.5 m, 1396.0 m, 1486.0 w, 1532.8 m, 1587.3 w, 1628.5 w, 3066.0 w cm⁻¹.

Crystal data, modification A: C₁₉H₁₄O₂ (274.30), monoclinic, space group P2₁/c, a = 7.6971 (4), b = 15.0236(6), c = 12.1445(7) Å, (β = 93.550(3)°, V = 1401.67(12) Å³, Z = 4, D_{c} = 1.300 Mg/m³, µ(Mo-Kα) = 0.71073 Å, T = 233(2) K, 2445 independent reflexes [Rᵢₙₜ = 0.0342], final R indices (199 parameters) for 2084 independent reflexes [I < 2σ(I)] are R1 = 0.0352, wR2 = 0.0892, GOF = 1.047.

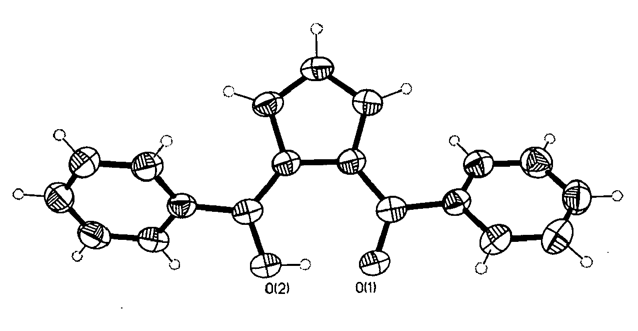

Crystal data, modification B: C₁₉H₁₄O₂ (274.30), monoclinic, space group *P*2₁/*c*, *a =* 10.5756(4), *b* = 15.5721(4), *c* = 17.4233(5) Å, *β*= 97.692(2)°, V= 2843.52(15) Å³, *Z* = 8, *D_{c}* = 1.281 Mg/m³, µ(Mo-Kα) = 0.71073 Å, *T* = 233(2) K, 5005 independent reflexes [*R*ᵢₙₜ = 0.0256], final R indices (396 parameters) for 3956 independent reflexes [*I*< 2σ(*I*)] are *R*1 = 0.0394, wR2 = 0.0939, GOF = 1.037.

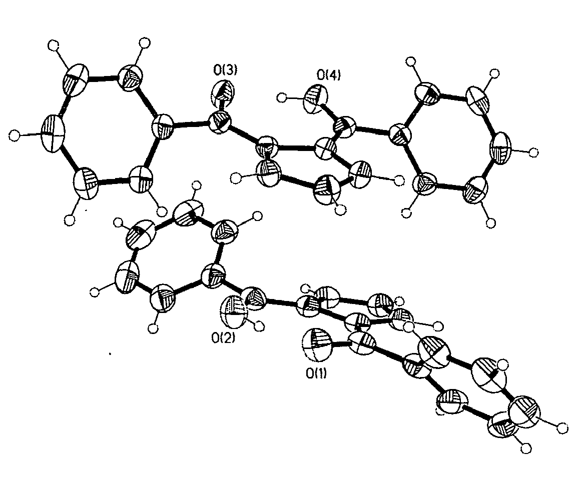

### Example 2

### Ligand 2: 2-(2,6-Dimethylphenyl)benzimidoyl-6-(2,6-dimethylphenyl)amino-6-phenylfulvene

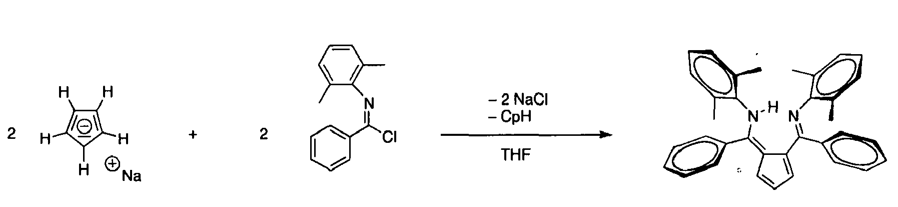

First a toluene stock solution (c = 15.07 g in 100 ml) of *N*-2,6-dimethylphenyl-benzimidoyl chloride was prepared from the corresponding benzamide and thionyl chloride following Boere's procedure (R.T. Boere, V. Klassen, G. Wolmershäuser, J. Chem. Soc., Dalton Trans. (1998) 4147). Second, a Schlenk vessel was charged with 6 ml THF, 1 ml (2 mmol) of a 2.0 molar sodium cyclopentadienide solution, and 3.5 ml (2.2 mmol) of the imidoyl solution from above. The mixture was stirred at room temperature for 30 minutes. Third, under protection from air, the solution was portioned into 4 pressure reaction vessels of a Paar Synthos 3000 microwave reactor. The microwave-assisted synthesis was performed under the following parameters: heating cycle 10 minutes up to 120 °C, then 90 minutes at 120 °C, maximum microwave power 400 W, cooling cycle 20 minutes.

Workup: The reaction vessels were carefully opened and their contents were hydrolyzed with ice/water. The four mixtures were combined, the pH was adjusted to neutral with ammonium chloride, THF was removed on a rotary evaporator, and the crude product was extracted into diethyl ether. The combined etheral layers were washed with water, the organic phase was dried with anhydrous sodium sulfate, and the product was purified by chromatography on neutral aluminum oxide with n-hexane/diethyl ether (v/v = 2/1) as eluent. The yellow product fractions were collected, reduced in volume on a rotary evaporator, and on standing the pure product crystallized in the form of large yellow crystals in 30% yield (145 mg).
m.p.: 252 °C. ¹H-NMR (CD₂Cl₂): 2.20 (s, 12H), 6.13 (t, 1H, J = 4 Hz), 6.42 (d, 2H, J = 4 Hz), 6.77-6.86 (m, 6H), 7.21-7.29 (m, 6H), 7.33-7.36 (m, 4H), enamine-H not observed, ppm. ¹³C-NMR (CD₂Cl₂): 19.0, 117.2, 123.5, 125.2, 127.3, 128.0, 128.6, 128.8, 131.7, 134.4, 137.5, 142.2, 166.5 ppm. MS (EI pos): 480.38 (M)⁺m/z. IR (ATR): 599.9 s, 665.5 s, 696.4 s, 757.8 s, 773.1 s, 889.5 s, 1027.5 m, 1049.1 m, 1088.8 m, 1160.3 m, 1305.4 m, 1348.2 m, 1439.3 m, 1471.3 m, 1517.5 m, 1641.0 m, 2853.8 w, 2917.2 w, 3018.5 w, 3265.4 w cm⁻¹.

Crystal data: C₃₅H₃₂N₂ (480.63), orthorhombic, space group *Iba2, a* = 11.3797(4), *b* = 13.7855(5), *c* = 17.0064(3) Å, *V =* 2667.88(14) Å³, *Z* = 4, *D_{c} =* 1.197 Mg/m³, µ(Mo-Kα) = 0.71073 Å, *T=* 233(2) K, 2466 independent reflexes [*R*ᵢₙₜ = 0.0223], final R indices (175 parameters) for 2339 independent reflexes [*I*< 2σ(*I*)] are *R*1 = 0.0322, wR2 = 0.0833, GOF = 1.046.

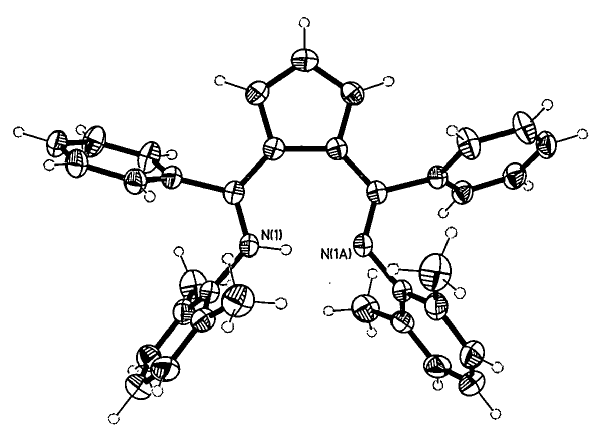

### Example 3

### Ligand 3: 2-(2,6-Diisopropylphenyl)benzimidoyl-6-(2,6-diisopropylphenyl)amino-6-phenylfulvene

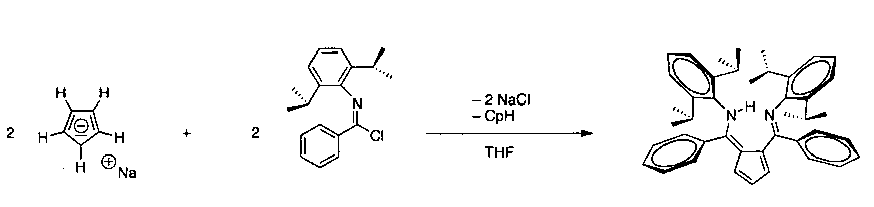

First a toluene stock solution (c = 15.29 g in 100 ml) of *N-*2,6-diisopropyllphenyl-benzimidoyl chloride was prepared from the corresponding benzamide and thionyl chloride following Boere's procedure (R.T. Boere, V. Klassen, G. Wolmershäuser, J. Chem. Soc., Dalton Trans. (1998) 4147). Second, a Schlenk vessel was charged with 6 ml THF, 2.8 ml (5.6 mmol) of a 2.0 molar sodium cyclopentadienide solution, and 11 ml (5.6 mmol) of the imidoyl solution from above. The mixture was stirred at room temperature for 30 minutes. Third, under protection from air, the solution was portioned into 4 pressure reaction vessels of a Paar Synthos 3000 microwave reactor. The microwave-assisted synthesis was performed under the following parameters: heating cycle 10 minutes up to 120 °C, then 90 minutes at 120 °C, maximum microwave power 400 W, cooling cycle 20 minutes.

Workup: The reaction vessels were carefully opened and their contents was hydrolyzed with ice/water. The four mixtures were combined, the pH was adjusted to neutral with ammonium chloride, THF was removed on a rotary evaporator, and the crude product was extracted into diethyl ether. The combined etheral layers were washed with water, the organic phase was dried with anhydrous sodium sulfate, and the product was purified by chromatography on neutral aluminum oxide with n-hexane/diethyl ether (v/v = 3/1) as eluent. The yellow product fractions were collected, reduced in volume on a rotary evaporator, and on standing the pure product crystallized in the form of large yellow crystals in 12% yield (200 mg).
m.p.: 208 °C. ¹H-NMR (CD₂Cl₂): 0.85 (d, J = 7 Hz), 1.05 (d, 12H, J = 7 Hz), 3.24 (sept, 4H, J = 7 Hz), 6.34 (t, 1H, J = 4 Hz), 6.76 (d, 2H, J = 4 Hz), 6.94-7.05 (m, 6H), 7.27-2.29 (m, 6H), 7.41-7.44 (m, 4H), 15.58 (s, 1H) ppm. ¹³C-NMR (CD₂Cl₂): 23.5, 24.8, 28.5, 117.6, 123.8, 124.3, 126.1, 127.3, 129.0, 130.4, 134.9, 137.1, 139.7, 141.5, 166.4 ppm. MS (FAB pos): 592.43 (M)⁺ m/z. IR (ATR): 504.3 m, 545.4 m, 582.6 m, 599.9 s, 666.5 m, 697.1 s, 740.6 s, 758.1 s, 773.5 s, 843.3 w, 889.3 m, 957.8 m, 1027.4 m, 1048.0 m, 1088.8 m, 1135.3 m, 1160.0 m, 1210.7 m, 1227.2 m, 1257.1 m, 1348.6 m, 1439.3 m, 1471.0 m, 1536.8 m, 1600.0 w, 2853.8 w, 2917.2 w, 2942.5 w, 3018.5 w, 3113.5 w, 3632.7 w cm⁻¹.

Crystal data: C₄₃H₄₈N₂ (592.83), monoclinic, space group *C*2/*c*, *a* = 13.8897(3), *b* = 11.0132(2), *c* = 24.4061(6) Å, β = 104.018(2)°, *V =* 3622.22(14) Å³, *Z =* 4, *D*_{c} *=* 1.087 Mg/m³, µ(Mo-Kα) = 0.71073 Å, *T =* 233(2) K, 3153 independent reflexes [*R*ᵢₙₜ = 0.0238], final R indices (209 parameters) for 2633 independent reflexes [*I* < 2σ(*I*)] are *R*1 = 0.0459, wR2 = 0.1200, GOF = 1.048.

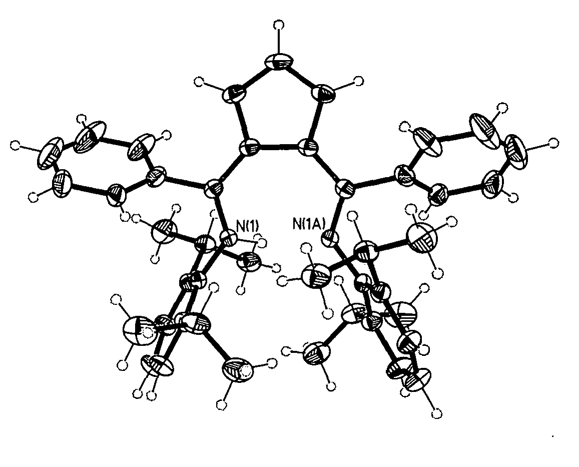

### Example 4

### Ligand 4: N,N'-Ethylenebis(2-benzoyl-6-hydroxy-6-phenylfulveneiminate)

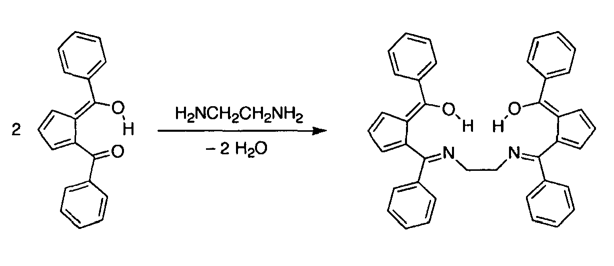

A small Schlenk vessel was charged with 363 mg (1.3 mmol) of ligand 1 and 5 ml of dry methanol. The suspension was gently heated and small portions of dry ethanol were added under stirring so that a clear yellow solution was obtained. To this solution, 44 µl (0.7 mmol) ethylenediamine was added and the mixture was refluxed for 2 hours, giving a yellow solution and a voluminous precipitate of the product. Precipitation was completed by cooling the mixture in an ice/water bath. Workup: The crude product was filtered off in air and washed with small portions of *n-*hexane. The solid yellow material was redissolved in dichloromethane and filtered through a short column of silica to remove a polar side product. After removal of dichloromethane on a rotary evaporator, the pure yellow product was dried in vacuo, affording 214 mg (57%) yellow powder.
m.p.: 238 °C. ¹H-NMR (CD₂Cl₂): 3.54 (m, 4H, J = 3 Hz), 6.15 (t, 2H, J = 4 Hz), 6.25 (d x d, 2H, J = 2 Hz), 7.01 (d x d, 2H, J = 2 Hz), 7.17-7.20 (m, 4H), 7.36-7.52 (m, 12H), 7.56 (t, 2H, J = 2 Hz), 7.59 (t, 2H, J = 2 Hz), 14.06 (s, 2H) ppm. ¹³C-NMR (CD₂Cl₂): 46.0,118.2,120.5,125.7,127.9,128.5,128.7,129.1, 129.8, 130.1, 134.0, 135.7, 139.7, 142.5, 166.8, 190.9 ppm. MS (FAB pos): 572.37 (M)⁺ m/z. IR (ATR): 593.4 m, 668.9 s, 697.5 s, 755.7 s, 771.5 m, 832.7 s, 893.4 m, 922.6 m, 933.7 m, 1021.7 m, 1046.9 m, 1079.0 m, 1133.7 m, 1239.5 m, 1261.6 m, 1279.4 m, 1314.7 m, 1345.4 m, 1359.8 m, 1396.1 m, 1438.3 s, 1471.8 m, 1489.7 m, 1541.6 s, 1579.9 m, 1632.8 s, 2853.8 w, 2920.3 w, 3053.3 w cm⁻¹.

Crystal data: C₄₀H₃₂N₂O₂ (572.68), monoclinic, space group *C2*/*c, a* = 24.0529(2), *b* = 6.4366(4), *c* = 20.2331(6) Å, *β* = 100.507(2)°, *V=* 3079.9(2) Å³, *Z* = 4, *D*_{c} = 1.235 Mg/m³, µ(Mo-Kα) = 0.71073 Å, *T=* 233(2) K, 2696 independent reflexes [*R*ᵢₙₜ = 0.0260], final R indices (204 parameters) for 2339 independent reflexes [*I* < 2σ(*I*)] are *R*1 = 0.0346, wR2 = 0.0872, GOF = 1.049.

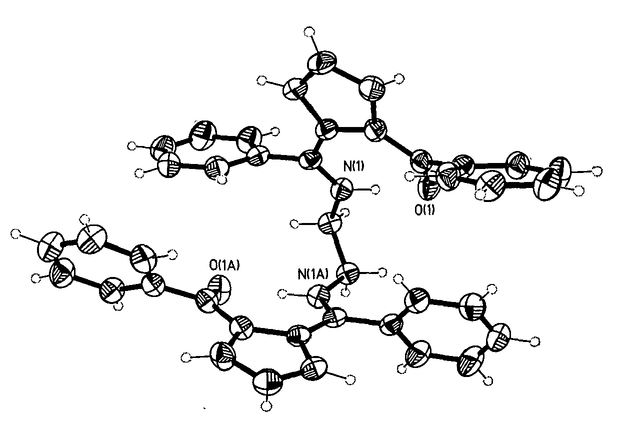

### Example 5

### Complex 1:

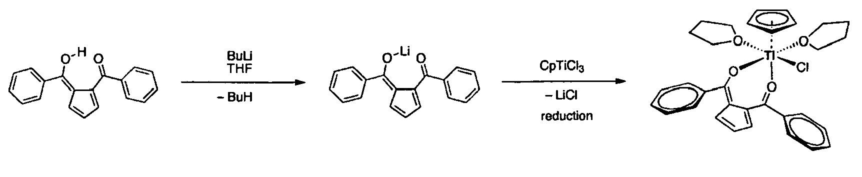

In a first Schlenk tube, a solution of 538 mg (1.96 mmol) ligand 1 in 15 ml THF was prepared. After cooling the yellow solution to -70 °C, 1.1 ml of a 2 molar n-butyl lithium solution in hexane (2.1 mmol) was added under stirring and the mixture was allowed to warm to room temperature. During deprotonation, the color of the solution changed from yellow to orange. Meanwhile, in a second Schlenk tube, a solution of 430 mg (1.96 mmol) cyclopentadienyltitanium trichloride in 15 ml of THF was prepared. The two solutions were combined, effecting an immediate change in color to dark brown. The mixture was stirred at ambient temperature over night.

Workup: Solvents and all volatile materials were removed on a vacuum line, the orange solid residue was taken up in dry dichloromethane, forming a green solution and a precipitate of colorless lithium chloride. The mixture was filtered through a Schlenk filter tube and the filtrate was evacuated to dryness on a vacuum line, resulting in 927 mg (79%) of the product as a dark green, paramagnetic, air-sensitive powder. The colour indicates a reduction of Ti(IV) to Ti(III).

### Example 6

### Complex 2:

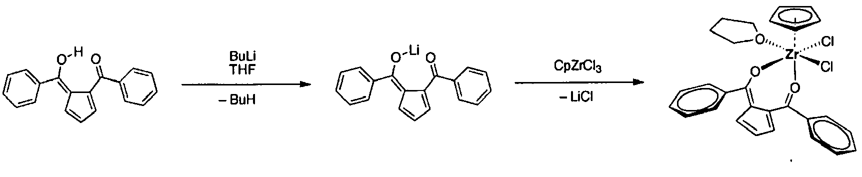

In a Schlenk tube a solution of 509 mg (1.9 mmol) of ligand 1 in 15 ml THF was prepared. After cooling the yellow solution to -70 °C, 1.1 ml of a 2 molar n-butyl lithium solution in hexane (2.1 mmol) was added under stirring and the mixture was allowed to warm to room temperature. During deprotonation, the color of the solution changed from yellow to orange. Meanwhile, in an inert atmosphere glove box, 506 mg (1.9 mmol) cyclopentadienylzirconium trichloride was weighed into a small Schlenk tube. This material was added to the solution of the deprotonated ligand and the mixture was stirred over night giving an orange solution. Workup: Solvents and all volatile materials were removed on a vacuum line, the orange solid residue was taken up in dry dichloromethane, forming an olive-green solution and a precipitate of colorless lithium chloride. The mixture was filtered in an inert atmosphere dry box through a Millipore syringe filter and the filtrate was evacuated to dryness on a vacuum line, resulting in 1.07 g (84%) of the product as an olive-green, air-sensitive powder. ,
¹H-NMR (CD_{Z}Cl₂): mixture of diastereoisomers: 1.74 (m, 4H, THF), 3.79 (m, 4H, THF), 6.48 (s), 6.51 (s), 7.04 - 7.29 (m), 7.40 (d), 7.52-7.68 (m), 7.80 - 7.82 (m) ppm. ¹³C-NMR (CD₂Cl₂): mixture of diastereoisomers: 190.0, 188.1, 187.7, 187.4, 185.3, 146.5, 146.2, 145.9, 145.8, 144.1, 140.1, 139.9, 139.7, 139.6, 138.8, 132.0, 131.1, 131.0, 130.9, 130.6, 130.2, 129.6, 129.5, 128.2, 127.9, 127.8, 127.6, 127.2, 124.6, 124.6, 121.9, 121.6, 121.2, 117.5, 117.3, 69.1, 25.2, 22.5 ppm.

### Example 7

### Complex 3:

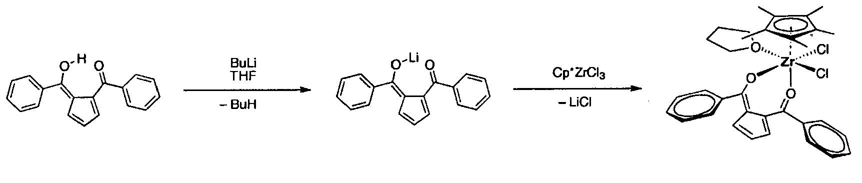

In a Schlenk tube a solution of 620 mg (2.3 mmol) of ligand 1 in 20 ml THF was prepared. After cooling the yellow solution to -70 °C, 1.38 ml of a 2 molar n-butyl lithium solution in hexane (2.8 mmol) was added under stirring and the mixture was allowed to warm to room temperature. During deprotonation, the color of the solution changed from yellow to orange. Meanwhile, in an inert atmosphere glove box, 765 mg (2.3 mmol) pentamethylcyclopentadienylzirconium trichloride was weighed into a small Schlenk tube. This material was dissolved in 15 ml THF and the resulting solution was added to the solution of the deprotonated ligand. The mixture was stirred over night giving an orange solution.

Workup: Solvents and all volatile materials were removed on a vacuum line, the orange solid residue was taken up in dry dichloromethane, forming an orange solution and a precipitate of colorless lithium chloride. The mixture was filtered in an inert atmosphere dry box through a Millipore syringe filter and the filtrate was evacuated to dryness on a vacuum line, resulting in 1.35 g (91 %) of the product as an orange, air-sensitive powder.
¹H-NMR (CD₂Cl₂): mixture of diastereoisomers: 1.76 (THF), 1.88, 1.92, 1.94, 1.98, 2.03, 2.05, 3.70 (THF), 6.41 (t), 6.85-7.92 (m) ppm. ¹³C-NMR (CD₂Cl₂): mixture of diastereoisomers: 11.3, 12.1, 12.4, 13.7, 18.8, 25.7, 68.5, 121.2, 121.9, 123.4, 124.0, 125.8, 126.4, 127.1, 127.8, 127.9, 128.3, 128.8, 129.4, 129.6, 129.8, 130.0, 130.1, 130.2, 131.1, 131.9, 140.3, 146.0, 188.0 ppm.

### Example 8

### Complex 4:

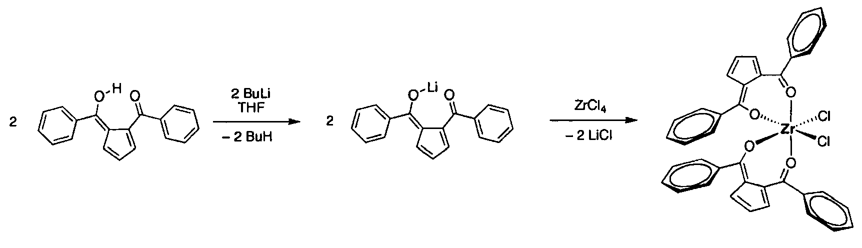

In a first Schlenk tube, a solution of 407 mg (1.5 mmol) ligand 1 in 15 ml THF was prepared. After cooling the yellow solution to -70 °C, 0.75 ml of a 2 molar n-butyl lithium solution in hexane (1.5 mmol) was added under stirring and the mixture was allowed to warm to room temperature. During deprotonation, the color of the solution changed from yellow to orange. Meanwhile, in a second Schlenk tube, a solution of 170 mg (0.7 mmol) zirconium tetrachloride in 15 ml of THF was prepared. The two solutions were combined and the orange mixture was stirred at ambient temperature over night.

Workup: Solvents and all volatile materials were removed on a vacuum line, the orange oily residue was taken up in dry dichloromethane, forming an orange solution and a precipitate of colorless lithium chloride. The mixture was filtered through a Schlenk filter tube and the filtrate was evacuated to dryness on a vacuum line, resulting in 479 mg (97%) of the product as an orange, air-sensitive powder.
¹H-NMR (CD₂Cl₂): 6.53 (t, 2H, J = 4 Hz), 7.31 (d, 4H, J = 4 Hz), 7.51-7.64 (m, 12H), 7.81-7.84 (m, 8H) ppm. ¹³C-NMR (CD₂Cl₂): 123.2, 124.4, 128.3, 129.8, 131.6, 137.7, 141.8, 185.4 ppm.

### Example 9

### Complex 5:

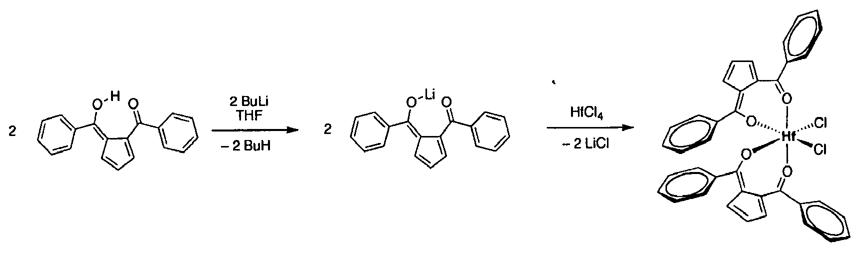

In a first Schlenk tube, a solution of 265 mg (1.0 mmol) ligand 1 in 15 ml THF was prepared. After cooling the yellow solution to -70 °C, 0.58 ml of a 2 molar n-butyl lithium solution in hexane (1.2 mmol) was added under stirring and the mixture was allowed to warm to room temperature. During deprotonation, the color of the solution changed from yellow to orange. Meanwhile, in a second Schlenk tube, a solution of 155 mg (0.48 mmol) hafnium tetrachloride in 15 ml of THF was prepared. The two solutions were combined and the orange mixture was stirred at ambient temperature over night.

Workup: Solvents and all volatile materials were removed on a vacuum line, the orange residue was taken up in dry dichloromethane, forming an orange solution and a precipitate of colorless lithium chloride. The mixture was filtered through a Schlenk filter tube and the filtrate was evacuated to dryness on a vacuum line, resulting in 355 mg (92%) of the product as an orange, air-sensitive powder.
¹H-NMR (CD₂Cl₂): 6.54 (t, 2H, J = 4 Hz), 7.31 (d, 4H, J = 4 Hz), 7.38-7.62 (m, 12H), 7.82-7.85 (m, 8H) ppm. ¹³C-NMR (CD₂Cl₂): 123.4, 124.7, 128.5, 130.1, 131.8, 133.1, 137.9, 185.6 ppm.

### Example 10

### Complex 6:

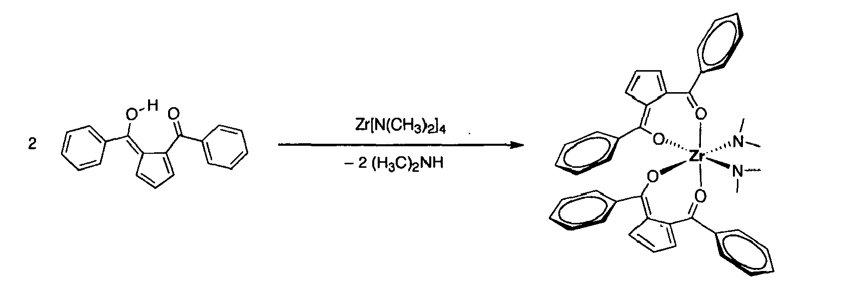

In a first Schlenk tube, a solution of 277 mg (1.0 mmol) ligand 1 in 15 ml toluene was prepared. In a second Schlenk tube, a solution of 135 mg (0.5 mmol) tetrakis(dimethylamino)zirconium in 15 ml of toluene was prepared. The two solutions were combined at -30 °C and the orange mixture was stirred at ambient temperature over night.

Workup: Solvents and all volatile materials were removed on a vacuum line, resulting in 394 mg (92%) of the product as an orange, air-sensitive powder.
¹H-NMR (CD₂Cl₂): mixture of two diastereoisomers: 2.34, 2.56, 3.03, 6.02-6.04 (m), 6.07-6.09 (m), 6.50 (t), 6.76-6.77 (m), 7.12-7.28 (m), 7.40-7.62 (m), 7.79-7.87 (m) ppm. ¹³C-NMR (CD₂Cl₂): 21.1, 44.3, 116.5, 119.1, 124.3, 125.1, 127.6, 127.7, 128.1, 128.9, 129.2, 129.6, 130.5, 130.7, 131.0, 131.3, 131.4, 133.7, 137.7, 141.2, 185.3, 190.6 ppm.

### Example 11

### Complex 7:

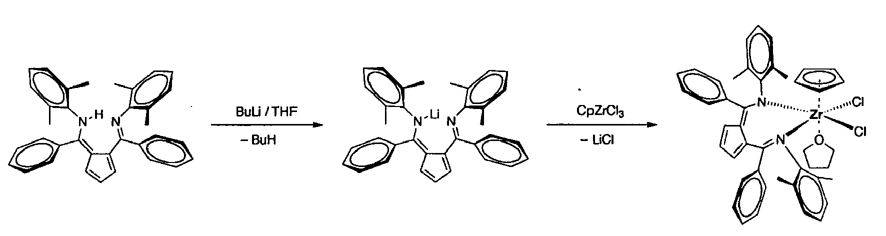

In a first Schlenk tube, a solution of 203 mg (0.4 mmol) ligand 2 in 30 ml THF was prepared. After cooling the yellow solution to -70 °C, 0.25 ml of a 2 molar n-butyl lithium solution in hexane (0.5 mmol) was added under stirring and the mixture was allowed to warm to room temperature. During deprotonation, the color of the solution changed from yellow to amber. Meanwhile, in a second Schlenk tube, a solution of 110 mg (0.4 mmol) cyclopentadienylzirconium trichloride in 15 ml of THF was prepared. The two solutions were combined and the amber mixture was stirred at ambient temperature over night.

Workup: Solvents and all volatile materials were removed on a vacuum line, the residue was taken up in dry dichloromethane, forming an orange solution and a precipitate of colorless lithium chloride. The mixture was filtered through a Schlenk filter tube and the filtrate was evacuated to dryness on a vacuum line, resulting in 300 mg (92%) of the product as an orange-red, air-sensitive powder.
¹H-NMR (CD₂Cl₂): 1.82 (THF), 2.17 (s, 12H), 3.67 (THF), 6.31-7.26 (m, 24 H) ppm.

### Example 12

### Complex 8:

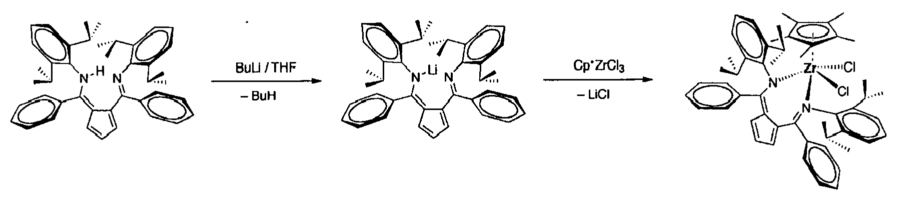

In a Schlenk tube a solution of 185 mg (0.3 mmol) of ligand 3 in 20 ml THF was prepared. After cooling the yellow solution to -70 °C, 0.2 ml of a 2 molar n-butyl lithium solution in hexane (0.4 mmol) was added under stirring and the mixture was allowed to warm to room temperature. During deprotonation, the color of the solution changed from yellow to orange. Meanwhile, in an inert atmosphere glove box, 100 mg (0.3 mmol) pentamethylcyclopentadienylzirconium trichloride was weighed into a small Schlenk tube. This material was dissolved in 15 ml THF and the resulting solution was added to the solution of the deprotonated ligand at -60 °C. The mixture was allowed to warm to room temperature and stirred further over night giving an orange solution.

Workup: Solvents and all volatile materials were removed on a vacuum line, the orange solid residue was taken up in dry dichloromethane, forming an orange solution and a precipitate of colorless lithium chloride. The mixture was filtered in an inert atmosphere dry box through a Millipore syringe filter and the filtrate was evacuated to dryness on a vacuum line, resulting in 254 mg (95%) of the product as an orange, air-sensitive powder.
¹H-NMR (CD₂Cl₂): 0.80 (d), 1.02 (d), 1.94-2.07 (m), 3.11-3.25 (sept), 6.20 (t), 6.58 (d), 6.88-7.02 (m), 7.26-7.29 (m), 7.34-7.38 (m) ppm. ¹³C-NMR (CD₂Cl₂): 12.0, 12.2, 14.0, 19.2, 23.1, 24.4, 28.6, 117.8, 123.8, 124.3, 126.0, 127.3, 128.9, 130.2, 135.0, 137.2, 139.7, 141.6, 166.4 ppm.

### Example 13

### Complex 9:

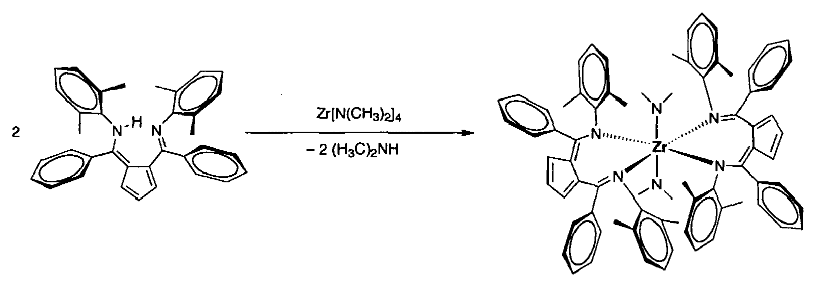

In a first Schlenk tube, a solution of 224 mg (0.47 mmol) ligand 2 in 15 ml toluene was prepared. In a second Schlenk tube, a solution of 65 mg (0.24 mmol) tetrakis(dimethylamino)zirconium in 15 ml of toluene was prepared. The two solutions were combined at -30 °C and the orange-brown mixture was stirred at ambient temperature over night.

Workup: Solvents and all volatile materials were removed on a vacuum line, resulting in 273 mg (100%) of the product as an orange, air-sensitive powder.
¹H-NMR (CD₂Cl₂): mixture of 2 diasteroisomers: 2.28 (s, 12H); 2.40, 2.71, 3.03 (3 x s, 6H); 6.21 (t, 1H, J = 4 Hz), 6.51 (d, 2H, J = 4 Hz), 6.83-6.92 (m, 6H), 7.20-7.34 (m, 10H), 7.41-7.45 (m, 4H) ppm. ¹³C-NMR (CD₂Cl₂): mixture of 2 diasteroisomers: 19.1, 19.3, 20.4, 21.5, 117.4, 122.7, 123.6, 125.3, 125.5, 127.3, 127.8, 128.1, 128.5, 128.6, 128.8, 129.3, 129.8, 131.8, 134.5, 137.6, 142.2, 166.6 ppm.

### Example 14

### Complex 10:

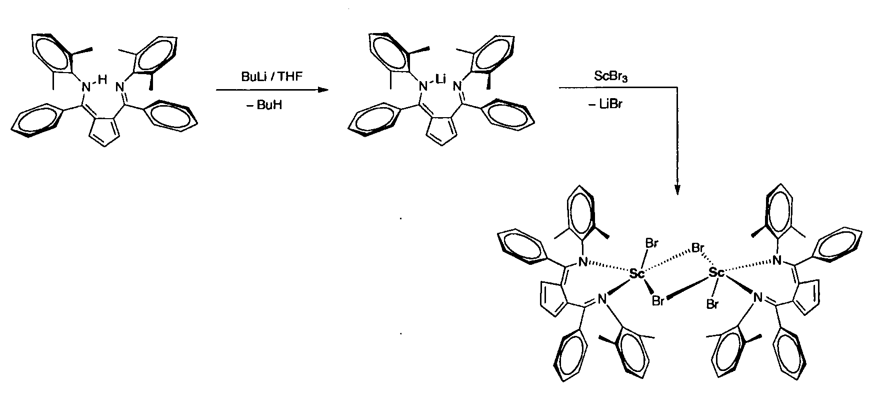

In a Schlenk tube a solution of 228 mg (0.47 mmol) of ligand 2 in 20 ml THF was prepared. After cooling the yellow solution to -70 °C, 0.28 ml of a 2 molar n-butyl lithium solution in hexane (0.56 mmol) was added under stirring and the mixture was allowed to warm to room temperature. During deprotonation, the color of the solution changed from yellow to orange. Meanwhile, in a second Schlenk tube, a suspension of 135 mg (0.47 mmol) scandium tribromide in 20 ml of THF was prepared and was added to the solution of the deprotonated ligand at -60 °C. The mixture was allowed to warm to room temperature and stirred further for 48 hours giving an orange solution.

Workup: Solvents and all volatile materials were removed on a vacuum line, the orange solid residue was taken up in dry dichloromethane, forming an orange solution and a precipitate of colorless lithium chloride. The mixture was filtered through a Schlenk filter tube and the filtrate was evacuated to dryness on a vacuum line, resulting in 231 mg (72%) of the product as a brown, air-sensitive powder.
¹H-NMR (CD₂Cl₂): 2.24 (s, 12H), 6.17 (t, 1H, J = 4 Hz), 6.47 (d, 2H, J = 4 Hz), 6.80-6.89 (m, 6H), 7.24-7.31 (m, 6H), 7.37-7.41 (m, 4H) ppm. ¹³C-NMR (CD₂Cl₂): 18.9, 117.1, 123.4, 125.1, 127.2, 127.9, 128.5, 128.7, 131.6, 134.3, 137.4, 142.1, 166.4 ppm.

### Example 15

### Complex 11:

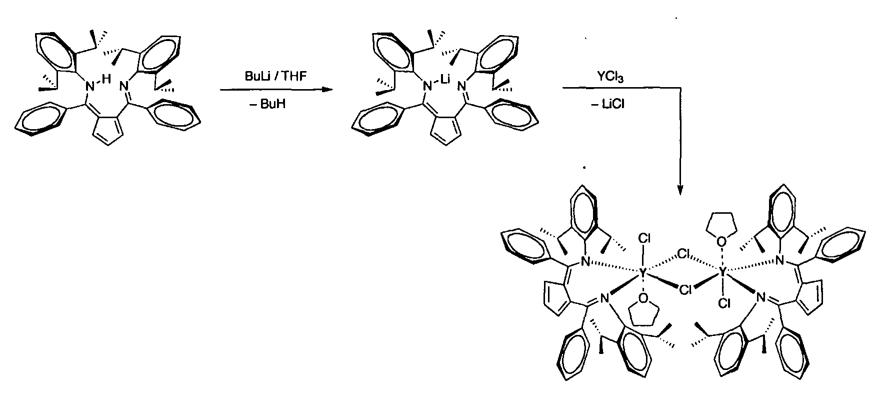

In a Schlenk tube a solution of 200 mg (0.3 mmol) of ligand 3 in 20 ml THF was prepared. After cooling the yellow solution to -70 °C, 0.18 ml of a 2 molar n-butyl lithium solution in hexane (0.36 mmol) was added under stirring and the mixture was allowed to warm to room temperature. During deprotonation, the color of the solution changed from yellow to orange. Meanwhile, in a second Schlenk tube, a solution of 66 mg (0.3 mmol) yttrium trichloride in 20 ml of THF was prepared and was added to the solution of the deprotonated ligand at -60 °C. The mixture was allowed to warm to room temperature and stirred further for 48 hours giving an orange solution.

Workup: Solvents and all volatile materials were removed on a vacuum line, the orange solid residue was taken up in dry dichloromethane, forming an orange solution and a precipitate of colorless lithium chloride. The mixture was filtered through a Schlenk filter tube and the filtrate was evacuated to dryness on a vacuum line, resulting in 163 mg (66%) of the product as an orange, air-sensitive powder.
¹H-NMR (CD₂Cl₂): 0.9 (d, 12H, J = 7 Hz), 1.01 (d, 12H, J = 7 Hz), 1.82 (br s, 4H), 3.16 (sept, 4H, J = 7 Hz), 3.71 (br s, 4H), 6.19 (t, 1H, J = 4Hz), 6.57 (d, 2H, J = 4 Hz), 6.87-7.01 (m, 6H), 7.25-7.36 (m, 10H) ppm. ¹³C-NMR (CD₂Cl₂): 22.7, 24.0, 25.4, 28.2, 67.8, 117.3, 123.3, 123.8, 125.6, 126.8, 128.5, 129.7, 134.5, 136.8, 139.3, 141.2, 165.9 ppm.

### Example 16

### Complex 12:

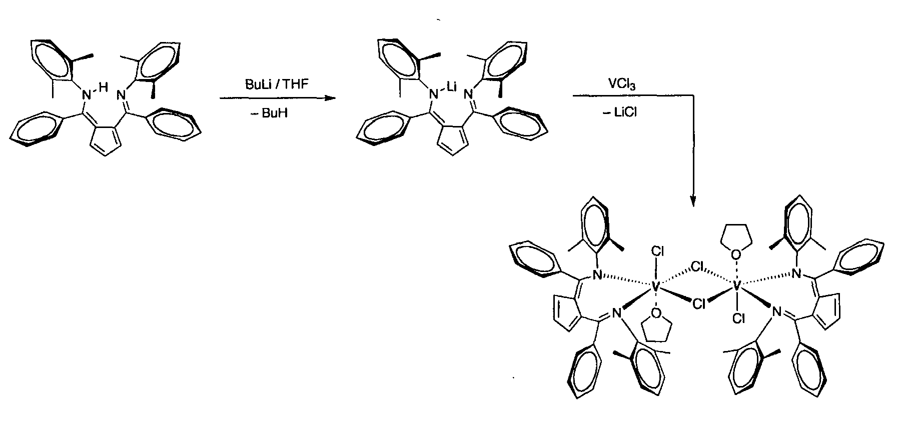

In a Schlenk tube a solution of 270 mg (0.56 mmol) of ligand 2 in 20 ml THF was prepared. After cooling the yellow solution to -70 °C, 0.33 ml of a 2 molar n-butyl lithium solution in hexane (0.67 mmol) was added under stirring and the mixture was allowed to .warm to room temperature. During deprotonation, the color of the solution changed from yellow to orange. Meanwhile, in a second Schlenk tube, a solution of 216 mg (0.56 mmol) tris(tetrahydrofuran)vanadium trichloride in 20 ml of THF was prepared and was added to the solution of the deprotonated ligand at -60°C. The mixture was allowed to warm to room temperature and stirred over night giving a dark brown solution. Workup: Solvents and all volatile materials were removed on a vacuum line, the orange solid residue was taken up in dry dichloromethane, forming a brown solution and a precipitate of colorless lithium chloride. The mixture was filtered through a Schlenk filter tube and the filtrate was evacuated to dryness on a vacuum line, resulting in 300 mg (80%) of the product as a brown, paramagnetic, air-sensitive powder.

### Example 17

### Complex 13:

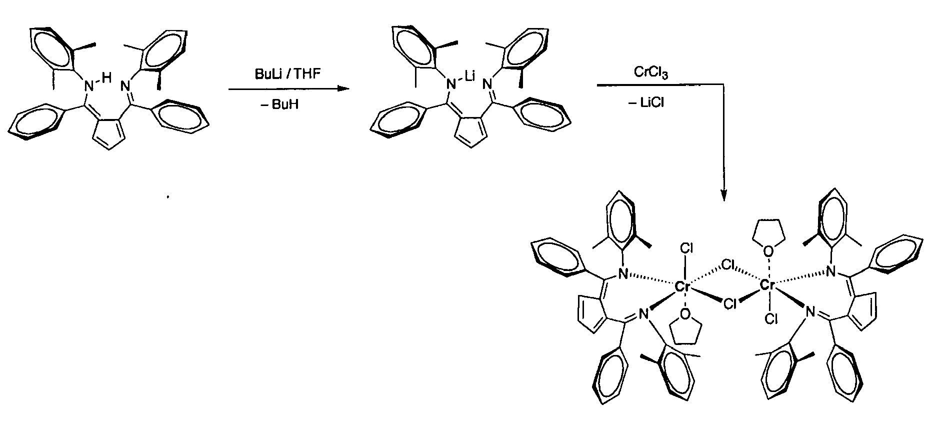

In a Schlenk tube a solution of 210 mg (0.44 mmol) of ligand 2 in 20 ml THF was prepared. After cooling the yellow solution to -70 °C, 0.26 ml of a 2 molar n-butyl lithium solution in hexane (0.53 mmol) was added under stirring and the mixture was allowed to warm to room temperature. During deprotonation, the color of the solution changed from yellow to orange. 70 mg (0.44 mmol) anhydrous chromium trichloride was added in one portion to the solution of the deprotonated ligand at room temperature. The mixture was stirred for 3 days giving an olive-brown solution. Workup: Solvents and all volatile materials were removed on a vacuum line, the green-brown solid residue was taken up in dry dichloromethane, forming a green-brown solution and a precipitate of colorless lithium chloride. The mixture was filtered through a Schlenk filter tube and the filtrate was evacuated to dryness on a vacuum line, resulting in 265 mg (90%) of the product as a green-brown, paramagnetic, air-sensitive powder.

### Example 18

### Complex 14:

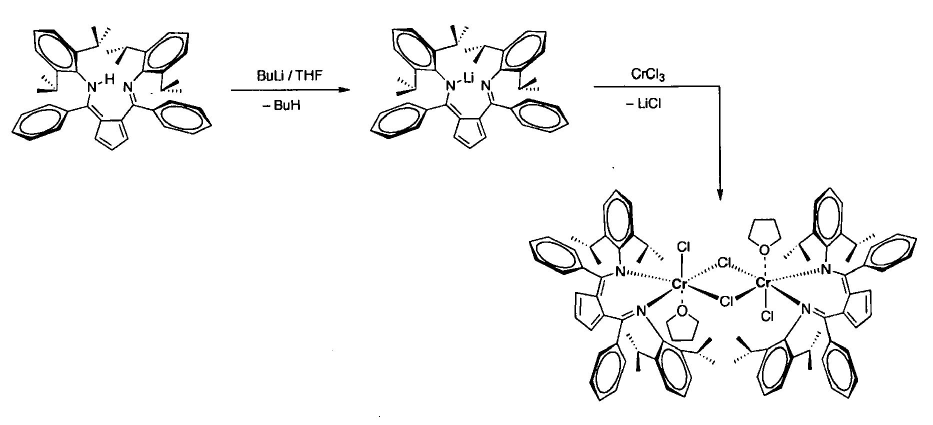

In a Schlenk tube a solution of 108 mg (0.18 mmol) of ligand 3 in 20 ml THF was prepared. After cooling the yellow solution to -70 °C, 0.12 ml of a 2 molar n-butyl lithium solution in hexane (0.3 mmol) was added under stirring and the mixture was allowed to warm to room temperature. During deprotonation, the color of the solution changed from yellow to orange. 120 mg (0.76 mmol) anhydrous chromium trichloride was added in one portion to the solution of the deprotonated ligand at room temperature. The mixture was stirred for 3 days giving an olive-brown solution.

Workup: Solvents and all volatile materials were removed on a vacuum line, the green-brown solid residue was taken up in dry dichloromethane, forming a green-brown solution and a precipitate of colorless lithium chloride. The mixture was filtered through a Schlenk filter tube and the filtrate was evacuated to dryness on a vacuum line, resulting in 130 mg (92%) of the product as a green-brown, paramagnetic, air-sensitive powder.

### Example 19

### Complex 15:

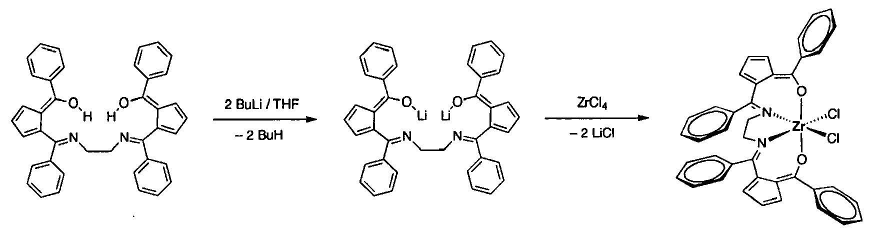

In a Schlenk tube a solution of 184 mg (0.32 mmol) of ligand 4 in 20 ml THF was prepared. After cooling the yellow solution to -70 °C, 0.35 ml of a 2 molar *n*-butyl lithium solution in hexane (0.7 mmol) was added under stirring and the mixture was allowed to warm to room temperature. During deprotonation, the color of the solution changed from yellow to orange. Meanwhile, in a second Schlenk tube, a solution of 75 mg (0.32 mmol) zirconium tetrachloride in 20 ml of THF was prepared and was added to the solution of the deprotonated ligand at -60 °C. The mixture was allowed to warm to room temperature and stirred over night giving an orange solution.

Workup: Solvents and all volatile materials were removed on a vacuum line, the orange solid residue was taken up in dry dichloromethane, forming an orange solution and a precipitate of colorless lithium chloride. The mixture was filtered through a Schlenk filter tube and the filtrate was evacuated to dryness on a vacuum line, resulting in 154 mg (66%) of the product as an orange, air-sensitive powder.
¹H-NMR (CD₂Cl₂): mixture of two diastereoisomers: 3.52-3.57 (m, 4H), 5.50-6.51 (m, 4H), 7.03-7.75 (m, 22H) ppm. ¹³C-NMR (CD₂Cl₂): mixture of two diastereoisomers: 43.7, 44.7, 44.9, 45.3, 46.1, 46.2, 107.1, 108.9, 118.3, 118.4, 119.4, 120.2, 120.3, 120.5, 120.7, 123.7, 125.4, 125.8, 126.4, 126.5, 127.2, 127.5, 127.9, 128.0, 128.1, 128.2, 128.3, 128.5, 128.6, 128.7, 128.8, 128.9, 129.1, 129.2, 129.3, 129.7, 129.9, 130.1, 130.2, 130.3, 130.5, 131.1, 132.1, 133.0, 133.8, 134.0, 134.6, 135.6, 135.8, 139.6, 139.8, 141.7, 142.2, 142.5, 146.1, 158.1, 159.6, 166.0, 166.5, 166.8, 190.8, 190.9 ppm.

### Example 20

### Complex 16:

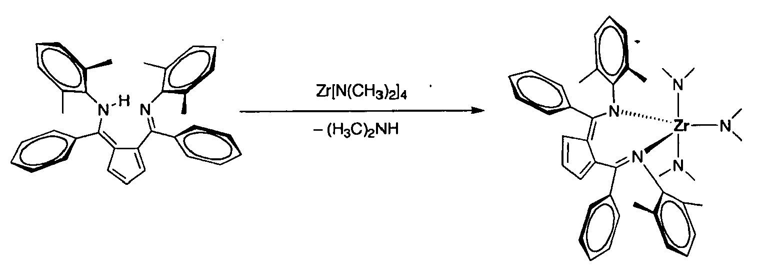

In a first Schlenk tube, a solution of 281 mg (0.58 mmol) ligand 2 in 15 ml toluene was prepared. In a second Schlenk tube, a solution of 160 mg (0.58 mmol) tetrakis(dimethylamino)zirconium in 15 ml of toluene was prepared. The two solutions were combined at -60 °C and the orange-brown mixture was stirred at ambient temperature over night, within this time the solution darkened from orange to brown.

Workup: Solvents and all volatile materials were removed on a vacuum line, resulting in 408 mg (100%) of the product as an orange, air-sensitive powder.
¹H-NMR (CD₂Cl₂): 2.21 (s, 12H); 2.34 (6H), 2.67 (4H); 2.75-3.45 (broad, 8H); 6.14 (t, 1H, J = 4 Hz), 6.43 (d, 2H, J = 4 Hz), 6.78 - 6.87 (m, 6H), 7.17 - 7.30 (m, 10H), 7.34 - 7.38 (m, 4H) ppm. ¹³C-NMR (CD₂Cl₂): 19.0, 19.2, 20.5, 21.5, 117.3, 122.8, 123.6, 125.3, 125.5, 127.3, 127.8, 128.0, 128.4, 128.6, 128.8, 129.3, 129.7, 131.8, 134.5, 137.6, 142.2, 166.6 ppm.

### Heterogenisation procedure.

The complexes were reacted for 0,5 h with MAO coactivator and then supported on Grace XPO-2485 silica during 1 h. The average particle size of the carrier was 20 µm, Al/M=200, (Zr 0,20 w-%), P.V. 1,4 mL/g SiO₂, and then dried at +25°C for 2 h. A typical heterogenisation procedure is shown below with complex 4.
1. Mix 0,66 mL of MAO (30 w-%) with 10,4 mg of complex 4 and with 0,04 mL of extra toluene.
2. Stir 30 min at +25°C
3. Add the solution from 2. to 0,5 g of Grace XPO-2485 during 10-15 min using a syringe.
4. Keep 1 h at +25°C with occasional stirring every 20 to 30 minute by hand. No magnetic stirring.
5. Evaporate the solvent off under Ar or N₂ flow at +25°C under 2 h. Orange/yellow powder.

The complexes were tested both as homogenous catalysts and as heterogeneous (supported) catalysts in ethylene polymerizations.

### Example 21

### Homogeneous Polymerisation results

Complexes were polymerized in homogeneous ethylene polymerization using MAO (30 % in toluene) as co-activator in all polymerizations. The polymerizations where carried out in 125 ml autoclave reactors.

The complex was contacted with the MAO in toluene for 1 h prior to polymerisation. 0,06 µmol (if not otherwise disclosed) of complex was used and the Al/Me ratio was 2000. The complex/MAO solution was added directly to the reactor in a glove box. Ethylene pressure was 10 bar, medium used was pentane 70 ml, stirring 250 rpm, and polymerisation time was 30 min.

Results are summarized in the Table 1.

### Example 22

### Heterogeneous polymerisation procedure:

Polymerisations were carried out in 125 ml autoclave reactors. The heterogeneous catalyst was added to a catalyst feeder, which then was attached to the reactor inside a glove box. Catalyst amount was 60-70 mg, no scavenger was used. Polymerisation temperature was 80°C, the ethylene partial pressure 10 bar, polymerization time 60 min and as medium was used iso-butane.

Results are presented in Table 1 below.

**Table 1**

| Complex no | Activity homog.polymerisation/ g pol/gMe*h | Activity heterog. polymerisation/ g pol/gcat*h |
|---|---|---|
| 1 | 14 | 3,3 |
| 2 | 57,1 | 40 |
| 3# | 24,4 | n.p. |
| 4 | 3,7 | 1,05 |
| 6## | 20,5 | n.p. |
| 7 | 0 | 24,8 |
| 8# | 48,7 | n.p. |
| 10 | 7,4 | 2,8 |
| 12 | 6,5 | 0 |
| 13 | 6,4 | 6,0 |
| 14 | 0 | 26,5 |
| 16## | 8,2 | n.p. |

| | | |
|---|---|---|
| # 0.09µmol complex was used ## 0,1 µmol complex was used | | |

## Claims

1. An olefin polymerisation catalyst comprising:
(I) a complex comprising a ligand of formula (I) 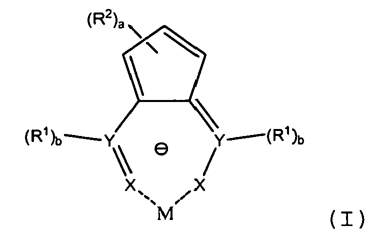 coordinated (as shown) to a metal ion M; and
(II) a cocatalyst; or the reaction product thereof;
wherein each X which may be the same or different is NR³ or O;
each Y which may be the same or different is C, S, P, S=O;
each R¹ which may be the same or different is hydrogen, an optionally substituted C₁₋₂₀ hydrocarbyl group, -CN, N(R⁴)₂, silyl, siloxy, -CF₃, -SH, -C(=NR⁴)NR⁴R⁴, -NR⁴C(=O)R⁴, -NR⁴C(=NR⁴)R⁴, -NR⁴C(=NR⁴)NR⁴R⁴, -NR⁴C(=O)OR⁴, -NR⁴C(=O)NR⁴R⁴, -NR⁴S(O)₂R⁴, -S(O)₂NR⁴R⁴, -C(=S)NR⁴R⁴, -OC(=O)R⁴, -OC(=O)NR⁴R⁴, -OR⁴, optionally substituted heterocyclyl, or optionally substituted heteroaryl;
each R² which may be the same or different is an optionally substituted C₁₋₂₀ hydrocarbyl group, halo, silyl, siloxy, nitro, -CN, N(R⁴)₂, -CF₃, -SH, C(=O)R⁴, OC(=O)R⁴, C(=O)OR⁴, -OC(=O)NR⁴R⁴, -OR⁴, optionally substituted heterocyclyl, or optionally substituted heteroaryl or two R² groups attached to adjacent ring atoms together form an optionally substituted 5- to 8-membered fused ring;
each R³ which may be the same or different is hydrogen, or an optionally substituted C₁₋₂₀ hydrocarbyl group;
each R⁴ which may be the same or different is a hydrogen, an optionally substituted C₁₋₂₀ hydrocarbyl group, or two R⁴ groups taken together form an optionally substituted 5- to 8-membered fused ring;
a is 0 to 3;
b is 1 unless the atom Y to which R¹ is attached is P wherein b is 2.

2. A catalyst as claimed in claim 1, wherein the complex is of formula:
(I)M(LIG)c;
(I)₂M(LIG)c; or
(I)₃M
wherein (I) represents a ligand of formula (I) as defined in claim 1, M represents a metal ion, LIG represents a σ or η ligand and c is 1 to 5.

3. A catalyst as claimed in claim 1 or 2, wherein the metal ion M is coordinated by an optionally substituted cyclopentadienyl, tetrahydroindenyl, indenyl, chloro, C₁₋₆ alkyl, benzyl or amido (-NR⁴)₂ ligand.

4. A catalyst as claimed in claim 1 or 2 wherein said complex comprises a structure of formula (VII) 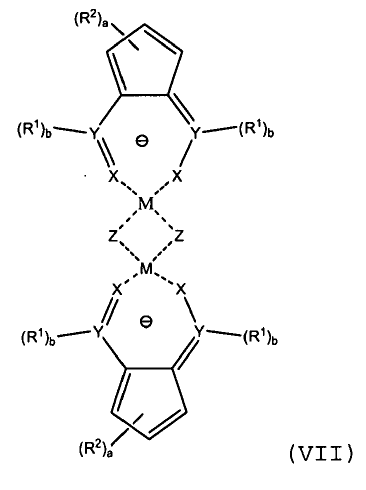 wherein R¹, R², X, Y, M, a and b are as defined in claim 1 and Z represents halo.

5. A catalyst as claimed in claim 1 or 2 wherein said complex comprises a ligand of formula (II) 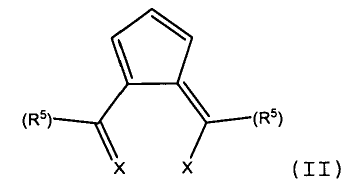 where R⁵ is an optionally substituted phenyl group and X is as defined in claim 1.

6. An olefin polymerisation catalyst comprising:
(I) a complex comprising a metal ion coordinated by at least one gamma diketonato and/or gamma diketiminato; and
(II) a cocatalyst; or the reaction product thereof.

7. Use in olefin polymerisation of a catalyst as claimed in any of claims 1 to 6.

8. A process for the polymerisation of at least one olefin comprising reacting said at least one olefin with a catalyst as claimed in any of claims 1 to 6.

9. A complex formed between a metal ion with a ligand of formula (III)
where R¹, R², and a are as defined in claim 1 and R^{3'} is as defined in claim 1 for R³ but does not represent hydrogen.

10. A complex as claimed in claim 6 of formula (III') 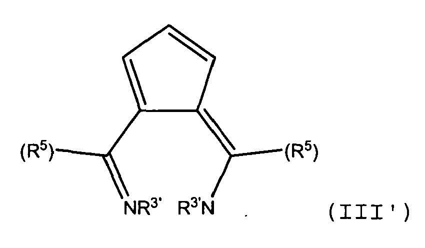 wherein R^{3'} and R⁵ are as defined in claim 9 and claim 5 respectively.

11. A complex formed between a metal ion M and a ligand of formula (I) 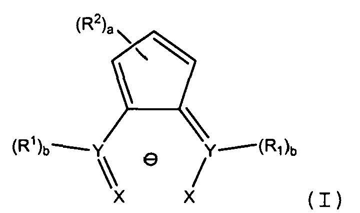 wherein R¹, R², X, Y, a and b as defined in claim 1 with the proviso that the ligand is not pentacarbomethoxycyclopentadienyl, or any of the following

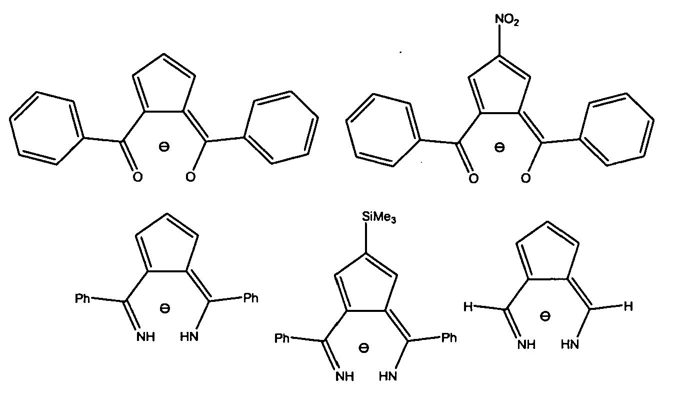

12. A complex comprising a metal ion M coordinated to a ligand of formula (IV) 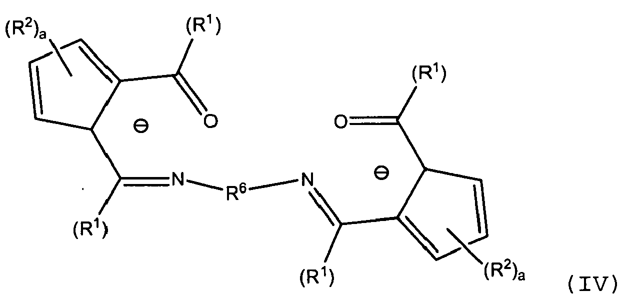 where R¹, R², and a are as defined in claim 1 and R⁶ is a linking group, e.g. an alkylene chain.

13. A complex comprising a structure of formula (VII) 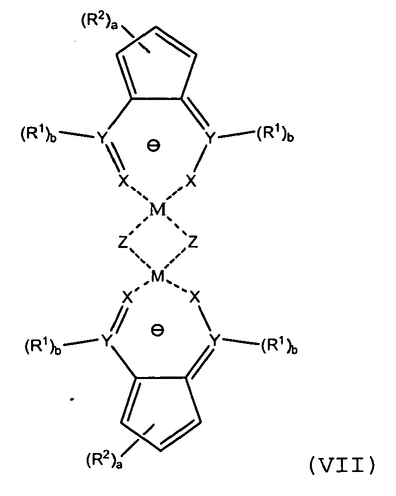 wherein R¹, R², X, Y, M, a and b are as defined in claim 1 and Z represents halo.

14. A process for the preparation of a tetradentate ligand comprising reacting a compound of formula (VII) 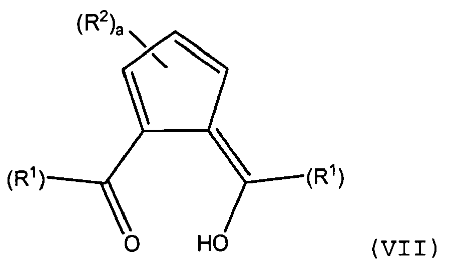 wherein R¹, R² and a are as defined in claim 1;
with a compound of formula NH₂R⁶NH₂ wherein R⁶ is a linking group

15. A process for the preparation of a compound of formula 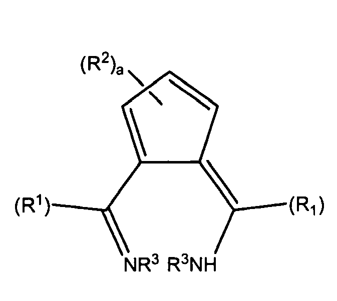 comprising reacting a compound of formula 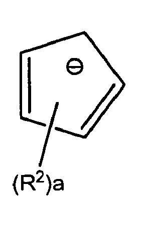 with a compound 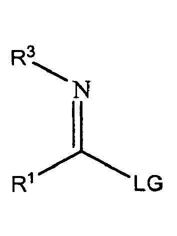 where LG is a leaving group, e.g. chloro.
